(19) Europäisches Patentamt
European Patent Office
Office européen des brevets

(11) Numéro de publication: 0 196 707 B1

(12) # FASCICULE DE BREVET EUROPEEN

(45) Date de publication du fascicule du brevet: **27.09.89**

(51) Int. Cl.⁴: **C 07 J 41/00,** C 07 J 43/00, C 07 J 51/00, C 07 J 71/00, A 61 K 31/585

(21) Numéro de dépôt: **86200441.3**

(22) Date de dépôt: **09.06.83**

(60) Numéro de publication de la demande initiale en application de l'article 76 CBE: **0097572**

(54) **Nouveaux 19-nor stéroides substitués en 11, en 17 et éventuellement en 2, leur préparation, leur application comme médicaments, les compositions les renfermant et les nouveaux intermédiaires obtenus.**

(30) Priorité: **11.06.82 FR 8210205**

(43) Date de publication de la demande: **08.10.86 Bulletin 86/41**

(45) Mention de la délivrance du brevet: **27.09.89 Bulletin 89/39**

(84) Etats contractants désignés: **AT BE CH DE FR GB IT LI LU NL SE**

(56) Documents cités:
**EP-A- 0 057 115**
**FR-A- 2 377 418**
**FR-A- 2 377 419**

(73) Titulaire: **ROUSSEL-UCLAF, 35, boulevard des Invalides, F-75007 Paris (FR)**

(72) Inventeur: **Teutsch, Jean-Georges, Résidence Lavoisier-Bât. 3 3, rue Lavoisier, F-93500-Pantin (FR)**
Inventeur: **Torelli, Vesperto, 5, rue de la Convention, F-94700 Maisons-Alfort (FR)**
Inventeur: **Deraedt, Roger, 23, Allée Jean-Baptiste Clément, F-93320 Pavillons-sous-Bois (FR)**
Inventeur: **Philibert, Daniel, 16, rue Chevalier, F-94210 La Varenne-Saint-Hilaire (FR)**

(74) Mandataire: **Bourgoin, André et al, Département des Brevets ROUSSEL UCLAF B.P. no 9, F-93230 Romainville (FR)**

EP 0 196 707 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux 19-nor stéroïdes substitués en 11β et éventuellement en 2, leur procédé de préparation, leur application comme médicaments, les compositions les renfermant et les nouveaux intermédiaires obtenus.

Dans les brevets français BF 2 377 418 et 2 377 419 sont décrits des produits stéroïdes comportant un substituant en position 11. Cependant les produits décrits dans ces brevets français ne comportent pas de cycle lactonique en position 17.

Dans le brevet européen EP 0 057 115 sont également décrits des produits comportant un substituant en position 11. Cependant le cycle lactonique que comportent les produits de la présente demande, bien que compris dans le champ de ce brevet, n'y est pas décrit expressement.

L'invention a pour objet les produits de formule générale ($I_D$):

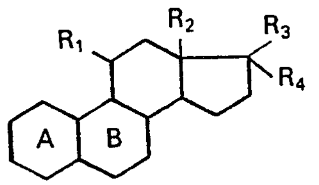

($I_D$)

dans laquelle $R_1$ représente un radical organique renfermant de 1 à 18 atomes de carbone et éventuellement un ou plusieurs hétéroatomes, l'atome immédiatement adjacent au carbone en 11 étant un atome de carbone, $R_2$ représente un radical hydrocarboné renfermant de 1 à 8 atomes de carbone, les cycles A et B ayant l'une des structures suivantes:

a) – Soit A et B représentent le groupement:

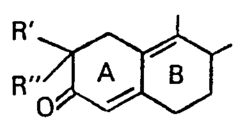

dans lequel R' et R" identiques ou différents représentent un atome d'hydrogène, un radical nitrile ou un radical alkyle ayant de 1 à 4 atomes de carbone étant entendu que l'un au moins des radicaux R' ou R" ne représente pas un atome d'hydrogène;

b) – Soit A et B représentent le groupement:

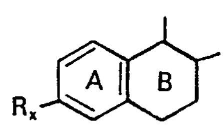

Rx représentant un atome d'hydrogène ou un groupement –ORe, dans lequel Re représente un atome d'hydrogène, un radical alkyle ayant de 1 à 6 atomes de carbone éventuellement substitué ou un radical acyle;

c) – Soit A et B représentent le groupement:

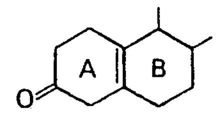

étant entendu que lorsque A et B représentent le groupement:

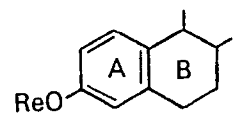

le radical $R_1$ contient au moins un atome d'azote, de phosphore ou de silicium, et que lorsque A et B représentent le groupement:

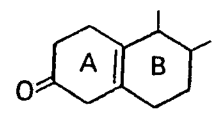

le radical $R_1$ ne représente pas un radical alkyle saturé linéaire, ainsi que les sels d'addition des produits de formule I avec les acides, et $R_3$ et $R_4$ forment ensemble le radical:

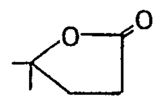

Le radical $R_1$ peut représenter un radical alkyle saturé ou insaturé linéaire ou ramifié ayant de 1 à 12 atomes de carbone.

Il s'agit alors de préference du radical méthyle, éthyle, propyle, isopropyle, butyle, isobutyle ou tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthyl heptyle, ou n-décyle. Il peut s'agir également des radicaux vinyle, isopropényle, allyle, 2-méthylallyle ou isobutényle.

Les radicaux précités peuvent être substitués. Parmi les substituants possibles on peut citer les radicaux thioalkyle tels que thiométhyle ou thioéthyle; $R_1$ peut également être substitué par un ou plusieurs atomes d'halogènes tels que fluor, chlore, brome, iode, ou par les radicaux amino substitués tels que diméthylamino, $R_1$ peut également représenter un radical aryle ou aralcoyle. Il s'agit alors de préference du radical phényle ou benzyle. Ces radicaux aromatiques peuvent être substitués en ortho, méta ou para par un ou plusieurs radicaux alkyles renfermant de préférence de 1 à 4 atomes de carbone; un ou plusieurs radicaux alkoxy ayant préférentiellement de 1 à 4 atomes de carbone tels que les radicaux méthoxy, éthoxy, propyloxy, isopropyloxy, butyloxy, isobutyloxy, tert-butyloxy; alkényloxy tel que vinyloxy ou allyloxy; un ou plusieurs atomes d'halogène, de préférence chlore ou fluor; par un ou

plusieurs radicaux choisis parmi les radicaux hydroxyle, trifluorométhyle, alkylthio ayant de 1 à 4 atomes de carbone éventuellement oxydé sous forme de sulfoxyde ou de sulfone tel que méthylthio, éthylthio; bien entendu les radicaux aryle ou aralkyle peuvent être substitués par une combinaison de ces différents radicaux tel que par exemple 3-fluoro, 4-diméthylamino phényle;

$R_1$ peut également représenter un radical aryle hétérocyclique éventuellement substitué par les différents radicaux envisagés ci-dessus. On peut citer les radicaux thiényle, furyle, isothiényle, isofuryle, thiazolyle, isothiazolyle, oxazolyle, isoxazolyle, thiadiazolyle, pyridinyle ou pipéridinyle et les hétérocycles connus de l'homme de métier;

$R_1$ peut également représenter un radical cycloalkyle tel que cyclopropyle, cyclobutyle, cyclopentyle ou cyclohexyle, cycloalkényle tel que cyclobutényle ou cyclopropényle;

$R_1$ représente également de préférence un radical comportant un noyau arylique substitué soit par une fonction amine éventuellement substituée par un ou deux radicaux alkyle ayant de 1 à 8 atomes de carbone, soit par un radical amine incorporé dans un hétérocycle comportant éventuellement un autre hétéroatome choisi dans le groupe formé par l'oxygène, l'azote et le soufre, tel que les radicaux morpholino ou pipéridinyle.

Le radical arylique est alors de préférence le noyau phényle.

Comme substituant sur le noyau arylique on peut également envisager un radical amino (substitué), alkyle tel que le radical diméthylamino méthyle, diméthylamino éthyle; un radical amino (substitué) alkyloxy tel que le radical diméthylamino éthyloxy.

On peut également citer les radicaux comportant un atome de silicium tel que le radical triméthylsilyl phényle.

Les radicaux précédemment cités comportant un atome d'azote peuvent être oxydés.

De manière générale, on préfère les produits dans lesquels le substituant $R_1$ comporte un hétéroatome de préférence l'azote ou le soufre.

Le radical $R_2$ représente de préférence un radical alkyle saturé, linéaire ou ramifié, renfermant de 1 à 4 atomes de carbone, par exemple un radical méthyle, éthyle, propyle ou butyle.

Préférentiellement $R_2$ représente un radical méthyle ou éthyle. Plus préférentiellement $R_2$ représente un radical méthyle.

Les valeurs de R' et R'' peuvent être choisies parmi les valeurs alkyles précédemment citées.

Lorsque Re représente un radical alkyle substitué, il s'agit de préférence d'un radical alkyle substitué par un radical dialkylamino tel que diméthylamino, diéthylamino ou méthyléthylamino.

L'invention s'étend naturellement aux sels d'addition avec les acides des composés de formule (I) salifiables, comme par exemple les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits de formule I on peut distinguer les produits dans lesquels $R_1$ représente un radical hydrocarboné renfermant de 1 à 18 atomes de carbone et contenant au moins un atome d'azote.

Parmi ces produits on peut citer tout spécialement les produits pour lesquels $R_1$ représente un radical alkyle primaire secondaire ou tertiaire, renfermant de 1 à 8 atomes de carbone, comportant un ou plusieurs hétéroatomes choisis dans le groupe constitué par l'oxygène, l'azote et le soufre, dont au moins un atome d'azote, ou substitué par un hétérocycle renfermant au moins un atome d'azote.

Par radical alkyle, on entend de préférence, les radicaux méthyle, éthyle, n-propyle, isopropyle, butyle, isobutyle, terbutyle, pentyle, hexyle, cyclopropyle, cyclobutyle, cyclopentyle ou cyclohexyle.

Par hétérocycle renfermant au moins un atome d'azote, on peut citer les radicaux 2,3- ou 4-pyridyle, les radicaux thiazolyle ou pipéridinyle.

On peut également citer comme produits de l'invention, les produits pour lesquels $R_1$ représente un radical hétérocyclique comportant au moins un atome d'azote, éventuellement substitué par un radical alkyle renfermant de 1 à 8 atomes de carbone. Les radicaux hétérocycliques sont de préférence ceux cités ci-dessus. Le radical alkyle préféré est alors un radical méthyle, éthyle ou propyle.

Parmi les produits préférés de l'invention on peut citer les produits de formule I telle que définie ci-dessus dans laquelle $R_1$ représente un radical aryle ou aralkyle portant une fonction amine

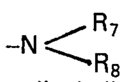

dans laquelle $R_7$ et $R_8$ représentent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisi dans le groupe constitué par l'oxygène, l'azote, le soufre et le silicium, dont au moins un atome d'azote, ou substitué par un hétérocycle comportant au moins un atome d'azote.

Les radicaux alkyle, aryle, aralkyle ou hétérocyclique sont de préférence ceux cités ci-dessus.

L'invention a tout spécialement pour objet les produits de formule I dans laquelle $R_1$ représente un radical 2, 3 ou 4-pyridyle,

un radical 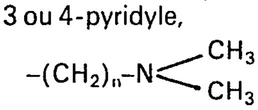 $(n \geqslant 3)$,

un radical:

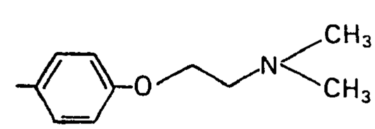

un radical

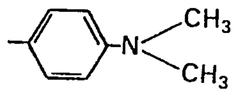

un radical

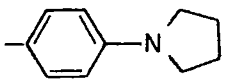

ou un radical

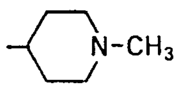

Une autre catégorie préférée de produits de l'invention est constituée par les produits de formule I dans laquelle $R_1$ représente un radical thiényle, furyle, cycloalkyle ayant de 3 à 6 atomes de carbone ou phényle éventuellement substitué par un ou plusieurs radicaux choisis parmi les radicaux hydroxy, halogène, trifluorométhyle, alkyle, alkoxy, alkylthio éventuellement oxydé sous forme de sulfoxyde ou de sulfone, étant entendu que les cycles A et B ne représentent pas le groupement:

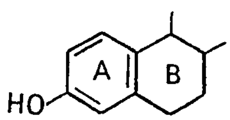

Les substituants préférés sont choisis dans les listes énoncées ci-dessus. Parmi les valeurs préférées de $R_1$ on peut alors citer le radical phényle substitué par un radical choisi dans le groupe formé par les radicaux chloro, fluoro, méthylthio, méthylsulfonyle, méthoxy, hydroxy et allyloxy.

Parmi les produits préférés figurent également les produits de formule I telle que définie précédemment dans laquelle les cycles A et B représentent le groupement

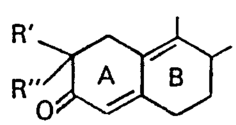

dans lequel R' et R'' sont tels que:

– soit R' et R'' identiques représentent chacun un radical méthyle ou un radical nitrile,

– soit l'un représente un atome d'hydrogène et l'autre représente un radical méthyle ou nitrile,

Parmi les produits préférés de l'invention, on peut donc naturellement citer les produits de formule $I_D$ telle que définie ci-dessus dans laquelle

$R_1$ représente le radical: 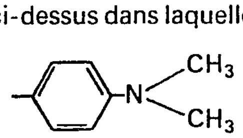

$R_2$ représente le radical $CH_3$ et 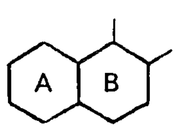

représente les radicaux:

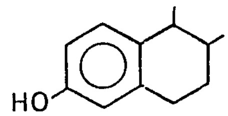 ; 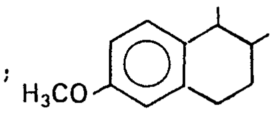 ;

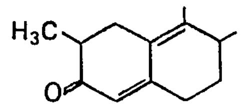 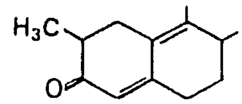

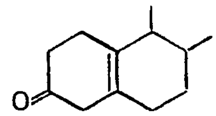 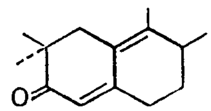

La présente invention a également pour objet un procédé de préparation des produits de formule générale $I_D$ telle que définie ci-dessus, caractérisé en ce que:

a) pour préparer les produits de formule $I_{DA}$:

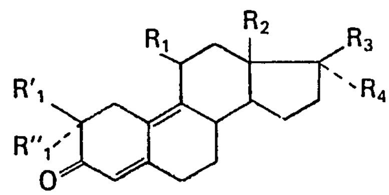 $(I_{DA})$

dans laquelle $R_1$, $R_2$, $R_3$ et $R_4$ conservent la même signification que ci-dessus et $R_1'$ et $R_1''$ sont tels que, ou bien $R_1'$ et $R_1''$ représentent chacun un radical alkyle ou bien l'un représente un atome d'hydrogène et l'autre représente un radical alkyle ou bien $R_1'$ et $R_1''$ représentent chacun un radical nitrile ou bien l'un représente un radical alkyle et l'autre un radical nitrile, on soumet un produit de formule $II_D$:

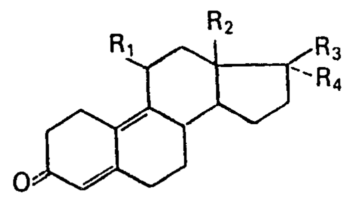 $(II_D)$

dans laquelle $R_1$, $R_2$, $R_3$ et $R_4$ conservent la même signification que ci-dessus d'abord, éventuellement à l'action d'un réactif de protection des groupements fonctionnels puis à l'action d'une base forte et ou bien d'un halogénure d'alkyle ou bien du cyanure de tosyle, ou bien d'abord à l'action d'un halogénure d'alkyle puis du cyanure de tosyle, puis élimine si nécessaire le groupement protecteur;

b) 1) pour préparer les produits de formule $I_{DB}$:

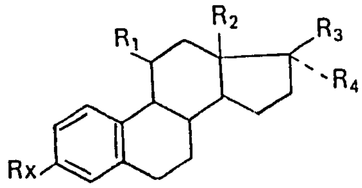

($I_{DB}$)

répondant à la formule $I_{DB1}$:

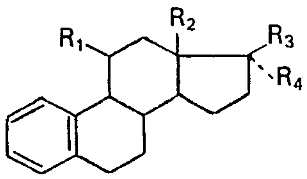

($I_{DB1}$)

dans laquelle $R_1$, $R_2$, $R_3$ et $R_4$ conservent la même signification que ci-dessus, on soumet un produit de formule $II_D$ à l'action d'un agent réducteur, pour obtenir un produit de formule $II_{D1}$:

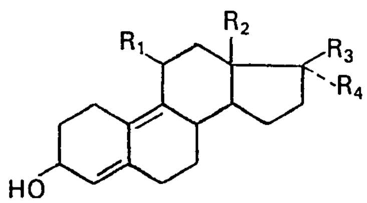

($II_{D1}$)

dans laquelle $R_1$, $R_2$, $R_3$ et $R_4$ conservent la même signification que ci-dessus, que l'on soumet à l'action d'un agent d'aromatisation acide;

b) 2) pour préparer les produits de formule $I_{DB}$ répondant à la formule $I_{DB2}$:

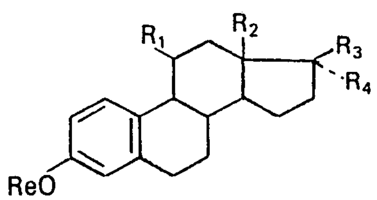

($I_{DB2}$)

dans laquelle $R_1$, $R_2$, Re $R_3$ et $R_4$ conservent la même signification que ci-dessus, on soumet un produit de formule $II_D$ à l'action d'un agent d'aromatisation puis de saponification, puis éventuellement à un réactif d'alkylation ou d'acylation;

c) pour préparer les produits de formule $I_{DC}$:

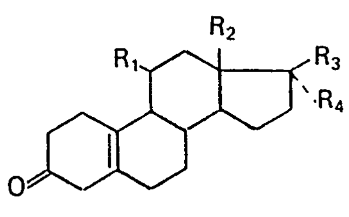

($I_{DC}$)

dans laquelle $R_1$, $R_2$, $R_3$ et $R_4$ conservent la même signification que ci-dessus, on soumet un produit de formule $II_D$ à l'action d'un agent réducteur;

d) pour préparer les produits de formule $I'_{DA}$

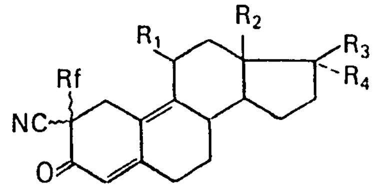

($I'_{DA}$)

dans laquelle Rf représente un atome d'hydrogène ou un radical alkyle et $R_1$, $R_2$, $R_3$ et $R_4$ conservent la même signification que ci-dessus, on fait agir une base sur les produits de formule $I_{DE}$ pour obtenir les produits de formule $I'_{DA}$ dans laquelle Rf représente un atome d'hydrogène et si désiré l'on soumet les produits ainsi obtenus à l'action d'une base forte et d'un halogénure d'alkyle pour obtenir les produits de formule $I'_{DA}$ dans laquelle Rf représente un radical alkyle.

Lorsque le produit de formule $II_D$ contient une ou plusieurs fonctions susceptibles de réagir par exemple avec l'halogénure d'alkyle utilisé, cette fonction est préférentiellement protégée à l'aide d'un groupement protecteur connu dans la littérature.

On utilise alors de préférence la protection par le groupement tétrahydropyrannyle obtenu par action sur le produit de formule $II_D$ du dihydropyranne.

La base forte que l'on utilise préférentiellement peut être un alcoolate alcalin tel que le tert-butylate de potassium. On peut également utiliser un amidure de métal alcalin tel que l'amidure de sodium ou de lithium éventuellement préparé in situ.

L'halogénure d'alkyle que l'on utilise est de préférence l'iodure, tel que l'iodure de méthyle.

De manière générale on obtient la gem-dialkylation en position 2 lorsqu'au moins deux équivalents de base forte sont utilisés, la réaction étant effectuée en présence d'un excès d'iodure d'alkyle.

La monoalkylation est effectuée au contraire lorsqu'un équivalent de base environ est utilisé.

En général, on obtient alors un mélange d'isomères $\alpha$ et $\beta$ qui peuvent être séparés par les techniques usuelles telles que la chromatographie.

L'addition de deux radicaux nitriles en position 2 est effectuée à l'aide de cyanure de tosyle dont la préparation est décrite dans Chem. Com. 440 (1968) et l'utilisation dans Tetrahedron Letters n° 50, 5011 (1981). L'addition d'abord d'un halogénure d'alkyle puis du cyanure de tosyle pour obtenir les produits dans lesquels l'un de $R_1'$ et $R_1''$ représente un radical alkyle et l'autre représente un radical nitrile est effectuée dans les conditions précédentes.

La déprotection éventuelle des fonctions bloquées est effectuée selon les méthodes classiques. Une hydrolyse acide peut par exemple être utilisée pour le tétrahydropyrannyle. On utilise alors l'acide chlorhydrique de préférence.

L'agent de réduction que l'on utilise pour préparer le produit de formule $II_{D1}$ est de préférence un borohydrure alcalin tel que le borohydrure de

sodium et l'on opère au sein d'un alcanol, tel que le méthanol ou l'éthanol.

L'agent d'aromatisation que l'on utilise de préférence pour préparer les produits de formule $IDB_1$ est un acide minéral tel que l'acide chlorhydrique ou l'acide sulfurique, ou encore un agent tel que le pentachlorure de phosphore, le tribromure de phosphore, l'oxychlorure de phosphore, ou encore un anhydride tel que l'anhydride acétique ou trifluoroacétique.

L'agent d'aromatisation que l'on utilise de préférence pour préparer les produits de formule $IDB_2$ est un halogénure d'acyle par exemple, le bromure d'acétyle ou l'anhydride acétique ou un mélange de ces produits.

L'agent de saponification utilisé est de préférence une base alcaline comme la soude ou la potasse, l'amidure de sodium, le tert-butylate de potassium ou l'acétylure de lithium dans l'éthylène diamine. La réaction a lieu, de préférence au sein d'un alcool inférieur tel que le méthanol ou l'éthanol.

L'alkylation éventuelle est effectuée selon les méthodes usuelles. On utilise un réactif d'alkylation qui est de préférence un halogénure d'alkyle tel que l'iodure d'alkyle ou le sulfate d'alkyle.

On utilise de préférence le sulfate de méthyle.

L'acylation est également réalisée selon les méthodes usuelles. On utilise de préférence un halogénure d'acyle.

L'agent réducteur que l'on utilise pour transformer les produits de formule $II_D$ en produits de formule $I_{DC}$ est de préférence un métal alcalin dans l'ammoniac liquide. On utilise de préférence le lithium mais le sodium peut également être envisagé.

Selon les quantités de métal employées, on peut également opérer des modifications à d'autres endroits de la molécule.

Lorsque seulement deux atomes de métal alcalin sont employés, la réduction 3-céto $\alpha$ 4,9 (10) en 3-céto $\alpha$ 5 (10) est pratiquement sélective.

Si une quantité supplémentaire de réducteur est employée on peut obtenir simultanément la réduction d'une fonction acétylénique en fonction trans oléfine, par exemple un radical 17$\alpha$ prop-1-ynyle peut être réduite en 17$\alpha$ prop-1-ényle.

La séparation des deux produits obtenus peut être réalisée par les méthodes classiques telle que la chromatographie.

L'invention a également pour objet les produits de formule $II_D$:

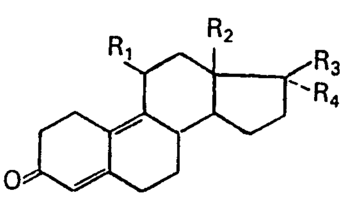

($II_D$)

dans laquelle $R_1$, $R_2$, $R_3$ et $R_4$ conservent la même signification qu'indiqué ci-dessus.

Parmi les produits de formule $II_D$ ou préféré les produits dans lesquels $R_2$ représente un radical méthyle et $R_1$ représente un radical:

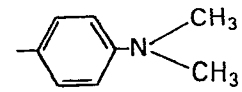

Les produits de formule $I_D$ ainsi que leurs sels d'addition avec les acides pharmaceutiquement acceptables sont des produits particulièrement intéressants du point de vue pharmacologique; ils possèdent en particulier une remarquable activité antiglucocorticoïde comme le montrent les résultats des tests joints en annexe.

L'étude des produits sur les récepteurs hormonaux a permis de mettre en évidence des activités progestomimétiques ou anti-progestomimétiques, androgènes ou anti-androgènes.

Les produits de formule $I_D$ ainsi que leurs sels d'addition avec les acides pharmaceutiquement acceptables peuvent donc être utilisés comme médicaments pour lutter principalement contre les effets secondaires des glucocorticoïdes, ils permettent de lutter également contre les troubles dus à une hypersécrétion de glucocorticoïdes et notamment contre le vieillissement en général et plus particulièrement contre l'hypertension, l'athérosclérose, l'ostéoporose, le diabète, l'obésité ainsi que la dépression de l'immunité et l'insomnie.

Les produits de formule $I_D$ ainsi que leurs sels d'addition avec les acides pharmaceutiquement acceptables, qui possèdent des propriétés anti-progestomimétiques peuvent être utilisés pour préparer des contraceptifs originaux ou comme agents d'interruption de grossesse.

Ces produits peuvent ainsi être utilisés comme inducteurs de règles chez la femme et plus généralement chez les animaux femelles à sang chaud.

Les produits sont alors administrés pendant les périodes où la progestérone joue un rôle physiologique essentiel, c'est-à-dire notamment pendant la phase lutéale du cycle, au moment de la nidation (ou implantation de l'embryon) et pendant la grossesse. Une méthode de contraception selon l'invention consiste à administrer à la femme un au moins des produits de formule $I_D$ pendant 1 à 5 jours se situant préférentiellement à la fin du cycle. Ces produits sont administrés alors préférentiellement par la voie orale ou in vagino mais ils peuvent également être utilisés par la voie parentérale.

Les produits peuvent également être utilisés par la voie endonasale.

Ces produits de formule $I_D$ possédant des propriétés antiprogestomimétiques peuvent également être utilisés contre les dérèglements hormonaux et, par ailleurs, ils peuvent présenter un intérêt dans le traitement des tumeurs hormonodépendantes.

Leurs actions sur les sécrétions hypophysaires rendent les produits utilisables dans la ménopause.

Ces produits peuvent également être utilisés dans la synchronisation de l'estrus chez les animaux d'élevage, en particulier les bovins et les ovins.

Les produits peuvent également être utilisés pour contrôler la fertilité des animaux familiers tels que chiens ou chats.

Certains produits de formule $I_D$ ainsi que leurs sels d'addition avec les acides pharmaceutiquement acceptables peuvent également présenter des propriétés progestomimétiques et peuvent ainsi être utilisés dans le traitement des aménorrhées, des dysménorrhées et des insuffisances lutéales.

Les produits de formule $I_D$ ainsi que leurs sels d'addition avec les acides pharmaceutiquement acceptables, qui présentent des propriétés anti-androgènes peuvent être utilisés dans le traitement des hypertrophies et du cancer de la prostate, de l'hyperandrogénie, de l'anémie, de l'hirsutisme et de l'acné. Ils peuvent également être utilisés pour la contraception chez l'homme.

Certains produits de formule $I_D$, ainsi que leurs sels d'addition avec les acides pharmaceutiquement acceptables peuvent également présenter des propriétés estrogènes.

Ils peuvent ainsi notamment être utilisés dans le traitement des troubles liés à une hypofolliculinie, par exemple les aménorrhées, les dysménorrhées, les avortements répétés, les troubles prémenstruels, ainsi que dans le traitement de la ménopause.

Certains produits de formule $I_D$, ainsi que leurs sels d'addition avec les acides pharmaceutiquement acceptables peuvent également présenter des propriétés anti estrogènes. Ils peuvent aussi être utilisés dans le traitement du carcinome mammaire et de ses métastases.

La posologie utile varie en fonction de l'affection à traiter et de la voie d'administration; elle peut varier par exemple de 10 mg à 1 g par jour chez l'adulte par voie orale.

Les nouveaux produits de formule $I_D$ et leurs sels, tels que définis ci-dessus peuvent être employés pour préparer des compositions pharmaceutiques renfermant, à titre de principe actif, l'un au moins desdits produits.

Les produits de formule $I_D$ et leurs sels sont utilisés par voie digestive, parentérale ou locale. Ils peuvent être prescrits sous forme de comprimés simples ou dragéifiés, de gélules, de granulés, de suppositoires, d'ovules, de préparations injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif au moins un produit de formule $I_D$.

Les produits de formule $II_D$ utilisés au départ du procédé de la présente invention peuvent être préparés comme suit:

On fait agir un produit de formule $(R_1)_2$ Cu Li, $R_1$ Mg Hal ou $R_1$ Li sur un produit de formule A

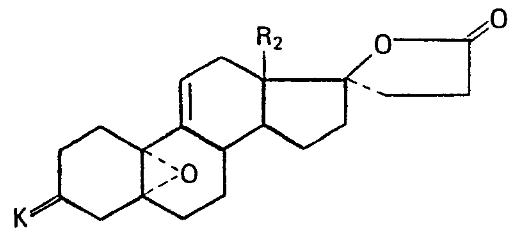

(A)

dans lequel K représente un groupement cétonique bloqué sous forme de cétal, de thiocétal, d'oxime, ou de méthyloxime pour obtenir un produit de formule B:

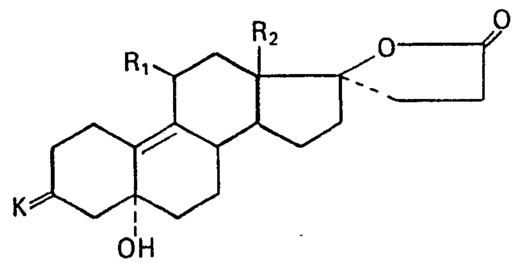

(B)

que l'on soumet à l'action d'un agent de déshydratation susceptible de libérer la ou les fonctions protégées pour obtenir un produit de formule $II_D$.

Comme agent de déshydratation on choisit de préférence une résine sulfonique à support de polystyrène ou un acide minéral tel que l'acide chlorhydrique ou sulfurique.

Les produits de formule A peuvent être préparés par action d'un oxydant tel que l'eau oxygénée en présence d'un catalyseur ou d'un peracide organique sur un produit de formule:

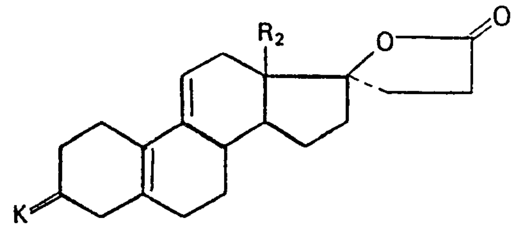

On trouvera dans la partie expérimentale ci-après des exemples de préparation de produits de départ de formule II.

En plus des exemples suivants qui illustrent l'invention sans toutefois la limiter, les produits suivants constituent des produits pouvant être obtenus dans le cadre de la présente invention.

| 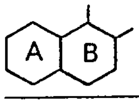 | $R_1$ | $R_2$ | $R_3$ | $R_4$ |
|---|---|---|---|---|
| 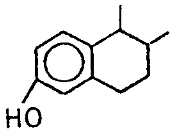 | 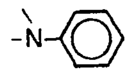 | | $CH_3$ | 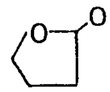 |
| 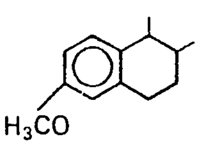 | 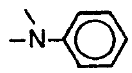 | | $CH_3$ | 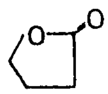 |
| 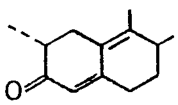 | 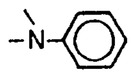 | | $CH_3$ | 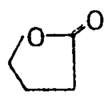 |
| 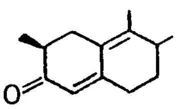 | 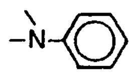 | | $CH_3$ | 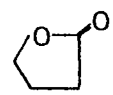 |
| 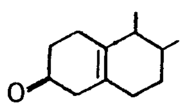 | 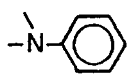 | | $CH_3$ | 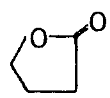 |
| 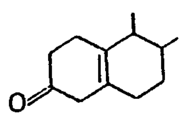 | 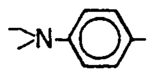 | | $CH_3$ | 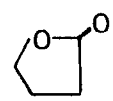 |
| 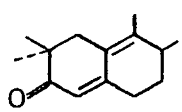 | 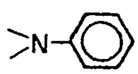 | | $CH_3$ | 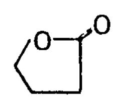 |

Exemple 1:

2,2-diméthyl 17β-hydroxy 11β-(4-diméthylamino phényl) 17α-(prop-1-ynyl) estra-4,9-dien-3-one

Stade A:

11β-(4-diméthylamino phényl) 17α-(prop-1-ynyl) 17β-[(2RS-tétrahydropyrannyl) oxy] estra-4,9-dièn-3-one.

On dissout 1,075 g de 17β-hydroxy 11β-(4-diméthylamino phényl) 17α-(prop-1-ynyl) estra-4,9-dièn-3-one dans 10 $cm^3$ de tétrahydrofuranne et 2 $cm^3$ de 3,4-dihydro 2H-pyranne, ajoute 570 mg d'acide paratoluène sulfonique et agite une heure à température ambiante. On ajoute 1 $cm^3$ de triéthylamine, dilue à l'acétate d'éthyle et lave avec une solution saturée de carbonate acide de sodium. On sèche, distille à sec sous pression réduite et obtient 2 g de produit brut sous forme d'huile jaune.

Stade B:

2,2-diméthyl 11β- (4-diméthylamino phényl) 17α- (prop-1-ynyl) 17β [(2RS-tétrahydro pyrannyl) oxy]estra- 4,9-dièn-3-one.

Le produit brut obtenu ci-dessus au stade A est dissous dans 15 $cm^3$ de tétrahydrofuranne anhydre. On refroidit vers −60 °C et ajoute sous agitation et en 5 à 6 minutes, alternativement par fractions de 1 $cm^3$, 3 $cm^3$ d'une solution 2M de tert-butylate de potassium dans le tétrahydrofuranne et une solution ajustée à 3 $cm^3$ de 0,38 $cm^3$ d'iodure de méthyle dans le tétrahydrofuranne. On agite 5 minutes après la fin de l'introduction, ajoute 1 $cm^3$ de triéthylamine, laisse revenir à température ambiante puis distille à sec.

Stade C:

2,2-diméthyl 17β-hydroxy 11β- (4-diméthylamino phényl) 17α- (prop-1-ynyl) estra-4,9-dièn-3-one

Le produit obtenu ci-dessus au stade A est dissous dans 10 $cm^3$ de méthanol. On ajoute 2,5 $cm^3$ d'acide chlorhydrique 5N. On laisse reposer 15 minutes à température ambiante puis dilue à l'eau et chasse le méthanol sous pression réduite. On ajoute 2,5 $cm^3$ d'ammoniaque concentrée, essore le produit précipité, et le lave à l'eau. On le redissout dans l'acétate d'éthyle, sèche la solution et distille à sec.

Le produit obtenu (1,5 g) est purifié par chromatographie sur silice (éluant: benzène-acétate d'éthyle 9/1). On obtient 990 mg de produit attendu cristallisé que l'on reprend dans l'heptane. On essore, lave, sèche et obtient 980 mg de produit purifié, F = 170–172 °C.

On obtient un produit pur en opérant comme suit: on joint au produit ci-dessus un échantillon de même qualité et dissout l'ensemble (2,2 g) dans du chlorure de méthylène. On filtre et distille à sec le filtrat. On dissout le filtrat au reflux de 10 $cm^3$ d'éther isopropylique. On refroidit, amorce la cristallisation par grattage et abandonne vers +5 °C. On essore les cristaux obtenus, les lave, les sèche et obtient 1,88 g de produit attendu, F = 171 °C. $(\alpha)_D$ = +183,5° ± 3,5 (c = 1% dans le chloroforme)

Analyse: $C_{31}H_{39}NO_2$

| | | | |
|---|---|---|---|
| Calculé: | C% 81,36 | H% 8,59 | N% 3,06 |
| Trouvé: | 81,1 | 8,8 | 3,2 |

Le 17β-hydroxy 11β- (4-diméthylamino phényl) 17α-(prop-1-ynyl) estra-4,9-dièn-3-one utilisé au départ de l'exemple 1 a été préparé dans la demande européenne n° 57 115.

Exemple 2:

11β-(4-diméthylamino phényl) 17α-(prop-1-ynyl) estra 1,3,5 (10)-triène 3,17-diol et 17β-acetoxy 11β-(4-diméthylamino phényl) 17α-(prop-1-ynyl) estra 1,3,5 (10)-trièn 3-ol

1) Aromatisation:

On introduit goutte à goutte à +5 °C 2 $cm^3$ d'anhydride acétique à 98% et 1 $cm^3$ de bromure d'acétyle dans une solution sous azote de 2 g de 17β-hydroxy 11β-(4-diméthyl amino phényl) 17α-(prop-1-ynyl) estra 4,9-dièn-3-one dans 20 $cm^3$ de chlorure de méthylène sec. On agite une heure à température ambiante puis ajoute 100 $cm^3$ de solution de carbonate acide de sodium. On agite 30 minutes, décante, extrait au chlorure de méthylène, sèche et amène à sec. On obtient 2,65 g de produit.

2) Saponification:

Le produit précédent (2,65 g) est dissous dans 25 $cm^3$ de méthanol puis on ajoute à température ambiante sous azote 2,6 $cm^3$ de lessive de soude. On agite 45 minutes, acidifie par 14 $cm^3$ d'acide chlorhydrique 2N puis alcalinise à l'ammoniaque. On extrait au chlorure de méthylène, lave à l'eau, sèche et amène à sec sous pression réduite. On obtient 2,3 g de produit que l'on chromatographie sur silice en éluant par un mélange chlorure de méthylène-acétate d'éthyle 8:2.

On obtient dans l'ordre 430 mg de 17β-acétoxy 11β-(4-diméthylamino phényl) 17α-(prop-1-ynyl) estra 1,3,5 (10) trièn-3-ol (Rf = 0,58) et 800 mg de 11β-(4-diméthylamino phényl) 17α-(prop-1-ynyl) estra 1,3,5 (10) trièn 3,17-diol (Rf = 0,36).

Le produit 17β acétoxy (430 mg) est purifié comme suit:

On le dissout dans 4 $cm^3$ de chlorure de méthylène au reflux, filtre, ajoute 4 $cm^3$ d'éther isopropylique, concentre à petit volume, refroidit, essore, lave à l'éther isopropylique puis sèche à 60 °C sous vide.

On obtient 370 mg de produit F = 236 °C, $(\alpha)_D$ = −194,5° ± 3°

Analyse: $C_{31}H_{37}NO_3$

| | | | |
|---|---|---|---|
| Calculé: | C% 78,95 | H% 7,91 | N% 2,97 |
| Trouvé: | 78,7 | 7,9 | 3,0 |

Le produit 3,17-diol est purifié comme suit:

On dissout 880 mg provenant de deux préparations similaires dans 12 $cm^3$ de chlorure de méthylène au reflux, filtre, ajoute 12 $cm^3$ d'éther isopropylique, concentre à 5 $cm^3$, glace, essore lave à l'éther isopropylique glacé puis sèche et obtient 770 mg de produit F = 246 °C, $(\alpha)_D$ = −188,5° ± 2,5°

Analyse: $C_{29}H_{35}NO_2$

| | | | |
|---|---|---|---|
| Calculé: | C% 81,08 | H% 8,21 | N% 3,26 |
| Trouvé: | 81,1 | 8,2 | 3,1 |

Exemple 3:

2-[(acétyloxy)méthylène] 11β-(4-diméthylamino phényl) 17β-hydroxy 17α-(prop-1-ynyl) estra 4,9-dièn-3-one.

Stade A:

17β-hydroxy 2-hydroxyméthylène 11β-(4-diméthyl amino phényl) 17α-(prop-1-ynyl) estra 4,9-dièn-3-one.

On lave trois fois 5 g d'hydrure de sodium en suspension à 55,5% dans l'huile par 50 $cm^3$ d'oxyde de diéthyle en éliminant le surnageant puis ajoute 100 $cm^3$ de benzène sec et 5 g de 17β-hydroxy 11β-(4-diméthylamino phényl) 17α-(prop-1-ynyl)estra 4,9-dièn-3-one. On ajoute ensuite goutte à goutte 9,4 $cm^3$ de formiate d'éthyle.

On agite 20 heures à température ambiante puis verse dans 200 $cm^3$ d'eau. On lave par 3 fois 50 $cm^3$ d'oxyde de diéthyle et réextrait par 2 fois 50 $cm^3$ d'eau. La phase organique est éliminée, les phases aqueuses rassemblées sont amenées à pH 3, 2 par addition de 70 $cm^3$ d'acide chlorhydrique 2N.

On revient à pH basique en rajoutant 40 $cm^3$ de solution saturée de carbonate acide de sodium.

On extrait par 100 $cm^3$ puis 3 fois 50 $cm^3$ d'oxyde de diéthyle, lave par 50 $cm^3$ de solution saturée de chlorure de sodium, sèche et porte à sec sous pression réduite. On obtient 5,2 g de produit brut utilisé tel quel pour le reste de la synthèse.

Stade B:

2-[(acétyloxy)méthylène] 11β-(4-diméthylamino phényl) 17β-hydroxy 17α-(prop-1-ynyl) estra 4,9 dièn-3-one.

On ajoute à +5 °C 0,2 $cm^3$ de pyridine puis 0,16 $cm^3$ de chlorure d'acétyle à une solution de 460 mg de produit obtenu au stade A dans 20 $cm^3$ de chloroforme sec. On laisse une heure au bain de glace, ajoute 10 $cm^3$ de solution aqueuse de carbonate acide de sodium, agite 5 minutes, décante, lave à l'eau, sèche puis amène à sec sous pression réduite.

On obtient 510 mg de produit brut que l'on dissout dans 10 $cm^3$ de chlorure de méthylène, ajoute 50 mg de charbon actif, filtre puis amène à sec. On obtient 490 mg de produit que l'on empâte dans l'éther de pétrole, lave ensuite à l'éther de pétrole puis à l'éther isopropylique (2 fois 5 $cm^3$). On sèche à 50 °C sous vide. On obtient 380 mg de produit attendu. $(\alpha)_D$ = +182° ± 3° (c=1% dans le chloroforme).

Analyse: $C_{32}H_{37}NO_4$

| | | | |
|---|---|---|---|
| Calculé: | C% 76,92 | H% 7,46 | N% 2,8 |
| Trouvé: | 77,0 | 7,6 | 2,6 |

Exemple 4:

11β-(4-diméthylamino phényl) 17β-hydroxy 2-(4-morpholinyl méthylène) 17α-(prop-1-ynyl) estra 4,9-dièn-3-one.

On ajoute 0,24 $cm^3$ de morpholine à une solution de 1,065 g de 17β-hydroxy 2-hydroxy méthylène 11β-(4-diméthylamino phényl) 17α-(prop-1-ynyl) estra 4,9 dièn-3-one obtenue comme à l'exemple 3 stade A, dans 10 $cm^3$ de méthanol. On chauffe une heure à 55 °C puis amène à sec à 50 °C sous pression réduite.

On obtient 1,25 g de produit brut que l'on chromatographie sur alumine (éluant: cyclohexane-acétone-triéthylamine 70/30/1) Rf = 0,4. On obtient 1,06 g de produit pur $(\alpha)_D$ = +263° ± 4,5° (c=0,7% dans le chloroforme).

Exemple 5:

11β-(4-diméthylamino phényl) 17β-hydroxy 2α-méthyl 17α-(prop-1-ynyl) estra 4,9-dièn-3-one, et 11β-(4-diméthylamino phényl) 17β-hydroxy 2β-méthyl 17α-(prop-1-ynyl) estra 4,9-dièn-3-one.

On introduit lentement à −70 °C sous argon une solution de 0,82 $cm^3$ de cyclohexyl isopropylamine dans 5 $cm^3$ de tétrahydrofurane dans un mélange de 3 $cm^3$ de solution 1,6 M de butyllithium dans l'hexane et de 5 $cm^3$ de tétrahydrofurane.

On laisse réagir dix minutes puis introduit 2,2 g

de 11β-(4-diméthylamino phényl) 17α-(prop-1-ynyl) 17β-[(2RS-tétrahydropyrannyl)oxy] estra 4,9-dièn-3-one préparé comme à l'exemple 1, stade A, en solution dans 8 $cm^3$ de tétrahydrofurane anhydre. On laisse réagir 10 minutes à −70 °C puis ajoute 0,5 $cm^3$ d'iodure de méthyle.

On laisse 30 minutes à −70 °C puis amène à température ambiante. On ajoute 1 $cm^3$ de triéthylamine puis dilue avec de l'acétate d'éthyle et lave à l'eau. Après séchage et évaporation du solvant on obtient 2,5 g de produit protégé en 17β par un radical tétrahydropyrannyle.

Ce produit est dissous dans 25 $cm^3$ de méthanol. On ajoute 4 $cm^3$ d'acide chlorhydrique à 50%. On laisse 2 heures à température ambiante puis dilue à l'eau et extrait au chlorure de méthylène. On lave à l'eau, sèche, évapore la phase organique sous pression réduite et obtient 2,04 g de produit attendu sous forme de mélange d'épimères 2-méthylés.

On chromatographie le mélange obtenu sur 300 g de silice avec un mélange éther-éther de pétrole (2/3).

On sépare 600 mg d'isomère 2β et 345 mg d'isomère 2α (et environ 280 mg de mélange).

On recristallise 1,1 g d'isomère 2β dans un mélange chlorure de méthylène-éther isopropylique et obtient 1,04 g de produit F = 211 °C.

On recristallise 703 mg d'isomère 2α dans le même mélange chlorure de méthylène-éther isopropylique et obtient 614 mg de produit purifié F = 204 °C. Une nouvelle recristallisation donne 560 mg de produit pur F = 205 °C.

Analyse: $C_{30}H_{37}NO_2$

| | | | |
|---|---|---|---|
| Calculé: | C% 81,22 | H% 8,40 | N% 3,15 |
| Trouvé | | | |
| isomère 2α: | 81,2 | 8,5 | 3,2 |
| isomère 2β: | 80,8 | 8,6 | 3,2 |

Exemple 6:

17β-hydroxy 11β-(3-méthoxyphényl) 17α-(prop-1-ynyl) ester-5(10) en-3-one et(E)17β-hydroxy 11β-(3-méthoxyphényl) 17α-(prop-1-ényl) estr 5(10) en-3-one.

On ajoute 20 $cm^3$ de tétrahydrofurane, 5 $cm^3$ de tert-butanol et 4,17 g de 11β-(3-méthoxy phényl) 17β-hydroxy 17α-(prop-1-ynyl) estra 4,9-dièn-3-one à 100 $cm^3$ d'ammoniac liquide à −55 °C. On ajoute à la solution obtenue 170 mg de lithium coupé en petits morceaux. Après deux heures à −55 °C on introduit lentement 50 $cm^3$ de solution aqueuse de chlorure d'ammonium, laisse revenir à température ambiante, extrait à l'acétate d'éthyle, lave à l'eau salée, sèche et amène à sec. On obtient 4,3 g d'une résine que l'on chromatographie sur 430 g de gel de silice en éluant par un mélange cyclohexane-acétate d'éthyle 7/3.

On obtient 380 mg de produit 17α prop-1-ényl, Rf = 0,27 et 2,65 g de produit 17α prop-1-ynyle, Rf = 0,25.

Constantes physiques:

Produit 17α prop-1-ényle $(\alpha)_D$ = +10,5° ± 1° (c=1,2% dans le chloroforme).

Analyse: $C_{28}H_{36}O_3$

| | | |
|---|---|---|
| Calculé: | C% 79,96 | H% 8,63 |
| Trouvé: | 79,8 | 8,9 |

Produit 17α prop-1-ynyle $(\alpha)_D$ = −30° ± 1,5 (c=1% dans le chloroforme);

Analyse: $C_{28}H_{34}O_3$

| | | |
|---|---|---|
| Calculé: | C% 80,34 | H% 8,19 |
| Trouvé: | 80,4 | 8,3 |

La 11β-(3-méthoxyphényl) 17β-hydroxy 17α (prop-1-ynyl)estra 4,9-dièn-3-one utilisée au départ de l'exemple 6 a été préparé comme suit:

a) 3,3-éthylène dioxy 11β-(3-méthyloxy phényl) 17α(prop-1-ynyl) estra 9-èn 5α, 17β-diol.

On agite à −20 °C pendant 15 minutes un mélange sous azote de 33,8 $cm^3$ de solution à 0,8 M de bromure de 3-méthyloxy phényl magnésium dans le tétrahydrofurane et de 285 mg de chlorure cuivreux puis ajoute en 15 minutes 3,3 g de 3,3-éthylène dioxy 5α,10α-époxy 17α(prop-1-ynyl) estr 9(11) en 17β-ol dans 33 $cm^3$ de tétrahydrofurane. Après quelques minutes on rajoute 33 $cm^3$ de tétrahydrofurane. Après une heure d'agitation à −20 °C on verse dans un mélange de 15 g de chlorure d'ammonium dans 200 $cm^3$ d'eau glacée. On agite une demi-heure, extrait trois fois à l'éther et lave à l'eau. On sèche et porte à sec sous pression réduite. On obtient 5,3 g de produit brut que l'on chromatographie sur ——— silice (éluant: chlorure de méthylène-acétone 95/5 à 1% de triéthylamine).

On isole 2,7 g de produit attendu, Rf = 0,30.

On recristallise 200 mg de produit dans le mélange chlorure de méthylène-éther isopropylique et obtient 165 mg de produit sous forme de cristaux blancs, F = 228 °C.

Analyse: $C_{30}H_{38}O_5$

| | | |
|---|---|---|
| Calculé: | C% 75,28 | H% 8,0 |
| Trouvé: | 75,3 | 8,1 |

b) 17β-hydroxy 11β-(3-méthyloxy phényl) 17α-(prop-1-ynyl)estra 4,9-dièn 3-one.

On porte à reflux sous agitation pendant une heure trente minutes un mélange de 2,5 g de 3,3-éthylène dioxy 11β-(3-méthyloxy phényl) 17α-(prop-1-ynyl) estra 9-èn 5α, 17β-diol dans 125 $cm^3$ d'éthanol à 95° et 2,5 g de résine Redex.

Après refroidissement on filtre la résine et rince abondamment avec de l'éthanol à 95°.

On porte le filtrat à sec sous pression réduite et obtient 2,38 g de produit brut que l'on chromatographie sur silice (éluant: éther de pétrole-acétate d'éthyle 3/2). On isole 1,75 g de produit amorphe que l'on recristallise dans un mélange éther-cyclohéxane. On essore, rince au cyclohexane, sèche sous vide et obtient 1,42 g de produit attendu, F = 164 °C.

Analyse: $C_{28}H_{32}O_3$

| | | |
|---|---|---|
| Calculé: | C% 80,73 | H% 7,74 |
| Trouvé: | 80,7 | 7,9 |

Exemple 7:

11β-(4-diméthylamino phényl) 17α-(prop-1-ynyl)isoxazolo [4,5-b] estra 4,9-dièn-17-ol.

On ajoute 350 mg de chlorhydrate d'hydroxylamine à une solution de 1,2 g de 17β-hydroxy 2-hydroxyméthylène 11β- (4-diméthylamino phényl) 17α-(prop-1-ynyl) estra 4,9-dièn 3-one dans 6 $cm^3$ de tert-butanol et porte 10 minutes au reflux.

On refroidit, dilue le mélange à l'eau et extrait au chlorure de méthylène. La phase organique lavée, séchée est évaporée sous pression réduite. On obtient 1,23 g de produit brut que l'on chromatographie sur 90 g de silice (éluant: éther de pétrole-éther 4/6). On obtient 755 mg de produit pur.

Exemple 8:

2-cyano 11β-(4-diméthylamino phényl) 17β-hydroxy 17α-(prop-1-ynyl) estra 4,9-dièn 3-one.

On fait barboter de l'azote à température ambiante pendant 15 minutes dans une solution de 1,2 g de produit préparé à l'exemple 7 dans 10 $cm^3$ de méthanol puis ajoute 3 $cm^3$ de potasse N.

On abandonne la solution sous azote à température ambiante pendant 3 heures. On dilue à l'eau, extrait au chlorure de méthylène, lave la phase organique à l'eau, sèche puis évapore sous pression réduite. On obtient 1,19 g de produit attendu que l'on chromatographie sur —— silice (éluant: éther-éther de pétrole 7/3). On obtient 685 mg de produit attendu.

Analyse: $C_{30}H_{34}O_2N_2$, 0,25 $H_2O$

| | | | |
|---|---|---|---|
| Calculé: | C% 78,48 | H% 7,57 | N% 6,10 |
| | | | $H_2O$% ≙ 1% |
| Trouvé: | 78,2 | 7,6 | 5,9 |
| | | | $H_2O$ ≙ 0,8% |

Exemple 9:

2,2-dicyano 11β-(4-diméthylamino phényl) 17β-hydroxy 17α-(prop-1-ynyl) estra 4,9-dièn-3-one.

On introduit sous argon 4,4 $cm^3$ d'une solution 1,6 M de butylithium dans l'hexane dans 10 $cm^3$ de tétrahydrofurane anhydre. On refroidit à −70 °C et ajoute une solution de 1,15 $cm^3$ de N-cyclohexyl isopropylamine dans 15 $cm^3$ de tétrahydrofurane anhydre.

On refroidit la solution ainsi obtenue à −70 °C et ajoute 1,290 g de 17β-hydroxy 11β-(4-diméthyl aminophényl) 17α-(prop-1-ynyl) estra 4,9-dièn-3-one en solution dans 12 $cm^3$ de tétrahydrofurane. On obtient la solution A.

On refroidit à −60 °C une solution de 2,55 g de cyanure de tosyle dans 15 $cm^3$ de tétrahydrofurane et ajoute goutte à goutte la solution A obtenue ci-dessus. On laisse réagir 40 minutes à −60 °C puis laisse revenir à température ambiante. On verse dans l'eau et extrait à l'acétate d'éthyle. La phase organique est lavée, séchée et évaporée sous vide. On obtient 2,85 g de produit que l'on chromatographie sur 80 g de silice (éluant: benzène-acétate d'éthyle 4/1). On obtient 1,06 g de produit attendu que l'on recristallise dans un mélange chlorure de méthylène-éther isopropylique. On obtient 774 mg de produit pur. F = 265 °C.

Analyse: $C_{31}H_{33}N_3O_2$

| | | | |
|---|---|---|---|
| Calculé: | C% 77,63 | H% 6,93 | N% 8,76 |
| Trouvé: | 77,6 | 7,0 | 8,7 |

Exemple 10:

17β-hydroxy 11β-(4-diméthylamino phényl) 17α-(prop-1-ynyl) estr 5(10) en-3-one.

On ajoute 15 $cm^3$ de tétrahydrofurane anhydre et 2,5 $cm^3$ de tert-butanol dans 40 $cm^3$ d'ammoniac liquide refroidi à −60 °C. On ajoute ensuite 2,15 g de 17β-hydroxy 11β-(4-diméthylamino phényl) 17α-(prop-1-ynyl) estra 4,9-dien-3-one. Après dissolution, on introduit 80 mg de lithium en 6 fractions en l'espace de 30 minutes. Après 30 minutes supplémentaires on retire le bain réfrigérant et fait couler lentement 40 $cm^3$ de solution aqueuse à 100 g/l de chlorure d'ammonium. On agite 30 minutes à température ambiante puis extrait à l'acétate d'éthyle. La phase organique est lavée avec une solution de chlorure d'ammonium, séchée et évaporée à sec. Les produits obtenus de 2,3 g sont chromatographiés sur 90 g de gel de silice (éluant: benzène-acétate d'éthyle 4/1). On obtient 1,67 g de produit attendu. Un échantillon analytique est préparé par nouvelle chromatographie sur silice (éluant: éther éthylique-heptane 2/1) $(\alpha)_D = -68,5° \pm 2°$ (c=1% dans le chloroforme).

Analyse: $C_{29}H_{37}NO_2$

| | | | |
|---|---|---|---|
| Calculé: | C% 80,7 | H% 8,64 | N% 3,25 |
| Trouvé: | 80,6 | 8,90 | 3,05 |

Exemple 11:

11β-(4-diméthylamino phényl) 3-méthoxy 17α-prop-1-ynyl estra 1,3,5(10) trien-17β-ol.

On ajoute en une seule fois 0,06 $cm^3$ de sulfate de méthyle à une solution maintenue à la température d'un bain de glace et constituée de 215 mg de 11β-(4-diméthylamino phényl) 17α(prop-1-ynyl) estra 1,3,5(10) trien 3,17-diol préparé à l'exemple 2 dans 6 $cm^3$ de soude 0,1 N et 6 $cm^3$ d'acétone. On supprime le bain de glace et agite 2 heures 30. On dilue par 30 $cm^3$ d'acétate d'éthyle, décante, lave à l'eau salée, sèche puis élimine les solvants et obtient 155 mg de produit que l'on chromatographie sur gel de silice (éluant: éther de pétrole-acétate d'éthyle 3/2) après dissolution dans le chlorure de méthylène.

On obtient 120 mg de produit attendu, Rf = 0,4

Exemple 12:

17β-acétoxy 11β-(4-diméthylamino phényl) 3-méthoxy 17α-(prop-1-ynyl) estra 1,3,5(10) triène.

On ajoute sous agitation 0,08 $cm^3$ de sulfate de méthyle à une solution maintenue sous azote de 330 mg de 17β-acétoxy 11β-(4-diméthylamino phényl) 17α-(prop-1-ynyl) estra 1,3,5(10) trien-3-ol préparé à l'exemple 2 dans 1,65 $cm^3$ d'acétone; 0,33 $cm^3$ d'eau et 0,42 $cm^3$ de soude 2N. On agite une heure puis dilue le milieu réactionnel avec de l'eau et extrait à l'acétate d'éthyle. On sèche la phase organique et évapore le solvant sous pression réduite.

On purifie le produit ainsi obtenu par chromatographie sur gel de silice (éluant: cyclohexane-acétate d'éthyle 4/1). On obtient 220 mg de produit attendu.

Exemple 13:
11β-[4-(2-diméthylaminoéthoxy) phényl] estra 1,3,5(10)trièn 3,17β-diol.
Stade A:
11β-[4-(2-diméthylaminoéthoxy] phényl) 3-hydroxy estra 1,3,5(10)trièn-17-one.

A 4 g de 11β-[4-(2-diméthylaminoéthoxy) phényl] estra 4,9-dien 3,17-dione dans 40 $cm^3$ de chlorure de méthylène à 0, +5 °C, on ajoute en 10 minutes, un mélange de 4 $cm^3$ d'anhydride acétique et 2 $cm^3$ de bromure d'acétyle, agite 2 heures en laissant revenir à température ambiante. On verse le mélange réactionnel dans 200 $cm^3$ d'une solution aqueuse saturée de bicarbonate de sodium et agite un quart d'heure. On extrait au chlorure de méthylène, lave les extraits organiques à l'eau, sèche et concentre à sec sous pression réduite. On obtient 4,33 g de produit que l'on dissout dans 40 $cm^3$ de méthanol, ajoute 4 $cm^3$ de lessive de soude, agite 1 heure 30 mn à température ambiante. On verse le mélange réactionnel dans 200 $cm^3$ d'eau, acidifie à pH ~ 2 — par addition de 30 $cm^3$ d'acide chlorhydrique 2N, puis amène à pH ~ 9 par addition de 5 $cm^3$ d'ammoniaque. On extrait au chlorure de méthylène, lave à l'eau, sèche et concentre à sec sous pression réduite. On chromatographie le résidu sur silice, élue par un mélange chlorure de méthylène-méthanol (9–1) et obtient 2,6 g de produit attendu.

Stade B:
11β-[4-(2-diméthylaminoéthoxy) phényl] estra 1,3,5(10)trièn 3,17β-diol.

On mélange 2,1 g du produit obtenu ci-dessus et 21 $cm^3$ de méthanol, ajoute par petites fractions 545 mg de borohydrure de sodium et agite à température ambiante pendant 1 heure. On verse dans 210 $cm^3$ d'un mélange glace et eau, extrait au chlorure de méthylène, lave à l'eau saturée en chlorure de sodium, sèche et concentre à sec sous pression réduite. On chromatographie le résidu sur silice en éluant par un mélange acétone-méthanol (8–2) et obtient 1,37 g de produit que l'on recristallise dans un mélange chlorure de méthylène-éther isopropylique. On isole 1,27 g de produit attendu. F = 130 °C. $(\alpha)_D = -46{,}5° \pm 1{,}5°$ (0,8% $CHCl_3$).

Le produit de départ du stade A a été préparé comme suit:

Stade a:
11β-[4-(2-diméthylaminoéthoxy) phényl] 3,3-éthylène dioxy 5α-hydroxy estr-9-ène 17-one.

Dans un mélange de 11,5 g de tournures de magnésium et 20 $cm^3$ de tétrahydrofuranne, on introduit, en 1 heure un quart et en maintenant la température à environ 35 °C, 97,4 g de 4-bromo 4-(2-diméthylaminoéthoxy) benzène dans 480 $cm^3$ de tétrahydrofuranne. On agite encore 1 heure et verse 380 $cm^3$ de cette solution dans une suspension contenant 23,5 g de complexe Cu Br, $(CH_3)_2S$ et 235 $cm^3$ de tétrahydrofuranne. Après un quart d'heure d'agitation à température ambiante, on ajoute en 20 minutes, 30 g de 3,3-éthylène dioxy 5α,10α-époxy estr-9(11)èn 17-one (décrite dans la demande européenne n° 57 115) dans 150 $cm^3$ de tétrahydrofuranne et laisse sous agitation à température ambiante pendant 16 heures. On verse le mélange réactionnel dans 3 litres de solution aqueuse saturée en chlorure d'ammonium. On extrait à l'éther, lave les solutions organiques avec une solution saturée de chlorure d'ammonium puis avec une solution saturée de chlorure de sodium, sèche et concentre à sec sous pression réduite. On obtient 66,7 g de produit brut que l'on chromatographie d'abord sur silice en éluant avec un mélange chloroforme-méthanol (9–1) à 1‰ de triéthylamine puis sur alumine en éluant avec un mélange benzène-acétate d'éthyle (8–2). On obtient 30,65 g de produit attendu.

Stade b:
11β-[4-(2-diméthylaminoéthoxy) phényl] estra 4,9-dièn 3,17-dione.

On agite 3 heures à température ambiante, 5 g de produit obtenu ci-dessus dans 100 $cm^3$ de méthanol et 15 $cm^3$ d'acide chlorhydrique 2N. On verse le mélange réactionnel dans 400 $cm^3$ d'éther et amène à pH alcalin par addition de 100 $cm^3$ de bicarbonate de sodium 0,5M. Après un quart d'heure d'agitation, on décante et extrait à l'éther, lave les phases organiques avec une solution saturée de chlorure de sodium, sèche et concentre à sec sous pression réduite. On obtient 3,90 g de produit que l'on empâte 3 fois avec un minimum d'éther isopropylique. On obtient 3,10 g de produit attendu. F = 206 °C.

Exemple 14:
11β-[4-(2-diméthylaminoéthoxy) phényl] estra 1,3,5(10)trièn 17β-ol.
Stade A:
11β-[4-(2-diméthylaminoéthoxy) phényl] estra 4,9-dièn 3,17β-diol (mélange d'isomère A et d'isomère B).

A 5 g de 11β-[4-(2-diméthylaminoéthoxy) phényl] estra 4,9-dièn 3,17-dione (obtenu au stade b de l'exemple 13) dans 100 $cm^3$ de méthanol, on ajoute 1,74 g de borohydrure de sodium par petites fractions en 20 minutes. On agite encore 1 heure à température ambiante et verse dans 750 $cm^3$ d'eau et glace et agite 30 minutes. On essore le précipité formé, le lave à l'eau jusqu'à neutralité, le sèche et obtient 4,6 g de produit, mélange des 2 isomères A et B. F ~ 110 °C.

Stade B:
11β-[4-(2-diméthylaminoéthoxy) phényl] estra 1,3,5(10)trièn 17β-ol.

On agite à température ambiante pendant 3 heures, 1 g de produit obtenu au stade A, 20 $cm^3$ de tétrahydrofuranne et 1 $cm^3$ d'acide chlorhydrique 6N. On verse le mélange réactionnel dans 200 $cm^3$ de solution aqueuse saturée de bicarbonate de sodium et extrait à l'acétate d'éthyle, puis lave avec une solution aqueuse saturée de chlorure de sodium, sèche et concentre à sec sous pression réduite. On chromatographie le résidu sur alumine en éluant par un mélange éther de pétrole (eb 60–80 °C)-acétone (8–2) et obtient 325 mg de produit que l'on dissout dans un mélange chlorure de méthylène-éther isopropylique (1–1), concentre lentement au demi et recueille 300,5 mg de produit attendu. F = 155 °C. $(\alpha)_D$ = −38,5° ± 2° (c=0,8% $CHCl_3$).

Exemple 15:
3-(2-diméthylamino) éthoxy 11β-phényl estra 1,3,5(10)trièn 17β-ol.
Stade A:
3-hydroxy 11β-phényl estra 1,3,5(10)trièn 17-one.

On refroidit à 0 °C, 3,76 g de 11β-phényl estra 4,9-dièn 3,17-dione dans 26,3 $cm^3$ de chlorure de méthylène et introduit goutte à goutte, sous agitation, un mélange contenant 3,75 $cm^3$ d'anhydride acétique et 1,9 $cm^3$ de bromure d'acétyle. On agite pendant 1 heure 15 minutes à température ambiante. On verse le milieu réactionnel, goutte à goutte, et sous agitation, dans 90 $cm^3$ d'une solution aqueuse saturée de bicarbonate. Après 15 minutes, on extrait au chlorure de méthylène, lave à l'eau, sèche, concentre à sec sous pression réduite. Au résidu, on ajoute 26,3 $cm^3$ de méthanol puis 18,8 $cm^3$ de lessive de soude et maintient l'agitation pendant 16 heures. On acidifie à ph ~ 1, en maintenant la température à ~ 20 °C, par addition de 40 $cm^3$ d'acide sulfurique dilué au 1/5ème et agite 20 minutes le produit cristallisé formé. On essore, lave par empâtage, avec 4 fois 25 $cm^3$ d'eau. On obtient 4,030 g de produit que l'on recristallise dans le chlorure de méthylène. On isole 3,01 g de produit pur. F = 290 °C. $(\alpha)_D$ = −9° ± 2° (c=0,5% $CHCl_3$).
Stade B:
11β-phényl estra 1,3,5(10)trièn 3,17β-diol.

On chauffe à 50 °C, sous agitation et sous atmosphère inerte 1 g du produit obtenu ci-dessus dans 10 $cm^3$ de méthanol et introduit en 10 minutes, 144 mg de borohydrure de sodium. Après 1 heure dans les mêmes conditions, on refroidit à +20 °C, amène le pH à 5 par addition, goutte à goutte, de 0,4 $cm^3$ d'acide acétique et agite 10 minutes. On verse le mélange réactionnel dans 30 $cm^3$ d'eau et glace, agite 30 minutes, essore, lave à l'eau par empâtage, sèche et obtient 896 mg de produit. F = 228 °C. $(\alpha)_D$ = −34° ± 2° (c=0,5% $CHCl_3$).
Stade C:
3-(2-diméthylaminoéthoxy) 11β-phényl estra 1,3,5(10)trièn 17β-ol.

On mélange 1,220 g de produit obtenu ci-dessus et 12,2 $cm^3$ d'éthanol à 95°. On ajoute en une seule fois 3,5 $cm^3$ d'une solution N de soude dans l'éthanol à 95° et chauffe à 60 °C. On ajoute en une fois, l'amine préparée à partir de 555 mg de chlorhydrate de diméthylamino 2-chloroéthane dissous dans 1,7 $cm^3$ d'éthanol à 95° puis neutralise par 3,85 $cm^3$ d'une solution N de soude dans l'éthanol à 95° fraichement préparée. On chauffe au reflux sous agitation et atmosphère inerte pendant 1 heure et 30 minutes. On refroidit à 20 °C, filtre le chlorure de sodium formé, l'empâte avec 10 $cm^3$ d'éthanol à 95°. On concentre à sec le filtrat. On agite le résidu avec 20 $cm^3$ de chlorure de méthylène et 20 $cm^3$ d'eau pendant 10 minutes, décante et extrait la phase aqueuse au chlorure de méthylène, lave les phases organiques à l'eau, sèche, concentre à sec sous pression réduite et obtient 1,261 g de produit brut. Après chromatographie sur silice élution par un mélange chloroforme-méthanol (6–4), on recueille 939 mg de produit attendu.

$(\alpha)_D$ = −32° ± 2° (c=0,7% $CHCl_3$).

Le produit de départ du stade A a été préparé comme suit:
Stade a:
3,3-éthylène dioxy 5α-hydroxy 11β-phényl estr-9-èn 17-one.

On introduit en 1 heure 10 minutes et en laissant la température s'élever jusqu'au reflux, 80 g de bromobenzène dans 400 $cm^3$ de tétrahydrofuranne, dans un mélange de 13 g de tournures de magnésium et 30 $cm^3$ de tétrahydrofuranne. On maintient l'agitation et laisse la température revenir à +20 °C. On refroidit à −25 °C, 330 $cm^3$ de la solution de bromure de phényl magnésien obtenue (0,85M), ajoute en une fois 4,165 g de chlorure cuivreux et agite 10 minutes à −25 °C. On introduit, goutte à goutte, en 20 minutes à −25 °C, 11,5 g de 3,3-éthylène dioxy 5α, 10α-époxy estr 9(11)èn 17-one dans 60 $cm^3$ de tétrahydrofuranne et maintient les conditions pendant 2 heures. On verse le mélange réactionnel dans 600 $cm^3$ d'eau et glace et 45 g de chlorure d'ammonium, agite 30 minutes et extrait à l'éther, lave à l'eau, sèche, concentre à sec sous pression réduite. On chromatographie le résidu sur silice, élue par un mélange benzène-acétate d'éthyle (6–4) à 1‰ de triéthylamine et obtient 7,86 g de produit attendu. F = 173 °C. $(\alpha)_D$ = +54,5° ± 1,5° (c=1% $CHCl_3$).
Stade b:
11β-phényl estra 4,9-dièn 3,17-dione.

On chauffe à 40 °C, 7,4 g de produit obtenu au stade a dans 225 $cm^3$ d'éthanol à 95 °C et introduit en une fois 7,4 g de Résine Redex CF. On chauffe au reflux pendant une heure sous agitation et sous gaz inerte, filtre la résine et la lave par empâtage 4 fois avec 20 $cm^3$ d'éthanol à 95°. On concentre à sec le filtrat sous pression réduite et obtient 6,5 g de résidu que l'on chromatographie sur silice, élue par un mélange de chloroforme-acétate d'éthyle (9–1) et obtient 486 mg de produit brut que l'on dissout au reflux dans un mélange de 12,5 $cm^3$ d'éther isopropylique et 3 $cm^3$ de chlorure de méthylène, filtre à chaud, concentre à faible volume et laisse cristalliser. On essore, lave à l'éther isopropylique et obtient 369,4 mg de produit attendu. F = 197 °C. $(\alpha)_D$ = +223° ± 3° (c=0,5% $CHCl_3$).

Etude pharmacologique des produits de l'invention.

I) Etude de l'activité des produits de l'invention sur les récepteurs hormonaux:

Récepteur minéralocorticoide du rein du rat:

Des rats mâles Sprague-Dawley EOPS, pesant 140 à 160 g, surrénalectomisés depuis 4 à 8 jours sont sacrifiés et leurs reins sont perfusés in situ avec 50 ml d'un tampon Tris 10 mM Saccharose 0,25 M, HCl pH 7,4. Les reins sont ensuite prélevés, décapsulés et homogénéisés à 0 °C à l'aide d'un Potter teflon-verre (1 g de tissu pour 3 ml de tampon). L'homogénat est centrifugé pendant 10 minutes à 800 g à 0 °C.

Afin d'éliminer la fixation de l'aldostérone tritiée sur le récepteur glucocorticoïde, le 11β, 17β dihydroxy 21-méthyl pregna 1,4,6-trien 20-yn 3-one stéroïde se fixant uniquement sur le récepteur glucocorticoïde est additionné au surnageant à la concentration finale de 10 $^{6}$M. Ce surnageant est ultracentrifugé à 105 000 g pendant 60 minutes à 0 °C. Des aliquotes du surnageant ainsi obtenu sont incubées à 0 °C avec une concentration constante (T) d'aldostérone tritiée en présence de concentrations croissantes (0–2500. $10^{-9}$M) d'aldostérone froide ou du produit froid à étudier. Après un temps (t) d'incubation, la concentration d'aldostérone tritiée liée (B) est mesurée par la technique d'adsorption au charbon-dextran.

Récepteur androgène de la prostate de rat:

Des rats mâles Sprague Dawley EOPS de 160 à 200 g sont castrés. 24 heures après la castration, les animaux sont sacrifiés, les prostates sont prélevées, pesées et homogénéisées à 0 °C à l'aide d'un Potter teflon-verre dans une solution tamponnée TS (Tris 10 mM, saccharose 0,25 M, HCl pH 7,4) (1 g de tissu pour 5 ml de TS). L'homogénat est ensuite ultracentrifugé (105 000 g pendant 60 minutes) à 0 °C. Des aliquotes du surnageant ainsi obtenu, sont incubées à 0 °C pendant un temps E d'incubation avec une concentration constante (T) de testostérone tritiée en présence de concentrations croissantes (0–1000. $10^{-9}$M), soit de testostérone froide, soit du produit à tester. La concentration de testostérone tritiée liée (B) est ensuite mesurée dans chaque incubat par la technique d'adsorption au charbon-dextran.

Récepteur Progestogène de l'utérus de lapine:

Des lapines impubères d'environ 1 kg reçoivent une application cutanée de 25 µg d'estradiol. 5 jours après ce traitement, les animaux sont sacrifiés, les utérus sont prélevés, pesés et homogénéisés à 0 °C, à l'aide d'un Potter teflon-verre dans une solution tamponnée TS (Tris 10 mM, saccharose 0,25 M, HCl pH 7,4) (1 g de tissu pour 50 ml de TS). L'homogénat est ensuite ultracentrifugé (105 000 g × 90 mn) à 0 °C. Des aliquotes du surnageant ainsi obtenu, sont incubées à 0 °C pendant un temps t, avec une concentration constante (T) de Produit R tritié (17,21-diméthyl 19-nor-4,9-pregnadiène-3,20-dione) en présence de concentrations croissantes (0–2500. 10 $^{9}$M) soit de R froid, soit de progestérone froide, soit du produit froid à tester. La concentration de R tritié lié (B) est ensuite mesurée dans chaque incubat par la technique d'adsorption au charbon dextran.

Récepteur glucocorticoïde du thymus de rat:

Des rats mâles Sprague Dawley EOPS de 160 à 200 g sont surrénalectomisés. 4 à 8 jours après cette ablation, les animaux sont sacrifiés, et les thymus sont prélevés et homogénéisés à 0 °C dans un tampon Tris 10 mM, saccharose 0,25M, dithiothreitol 2 mM, HCl pH 7,4, à l'aide d'un Potter polytétrafluoroéthylène-verre (1 g de tissu pour 10 ml de TS). L'homogénat est ensuite ultracentrifugé (105 000 g × 90 mn) à 0 °C. Des aliquotes du surnageant ainsi obtenu, sont incubées à 0 °C pendant un temps (t) avec une concentration constante (T) de dexaméthasone tritiée en présence de concentrations croissantes (0–2500.10 $^{9}$M) soit de dexaméthasone froide, soit du produit froid à tester. La concentration de la dexaméthasone tritiée liée (B) est ensuite mesurée dans chaque incubat par la technique d'adsorption au charbon-dextran.

Récepteur estrogène de l'utérus de souris:

Des souris femelles impubères âgées de 18 à 21 jours sont sacrifiées, les utérus sont prélevés puis homogénéisés à 0 °C à l'aide d'un Potter teflon-verre dans une solution tamponnée TS (Tris 10 mM, saccharose 0,25 M, HCl pH 7,4 (1 g de tissu pour 25 ml de TS). L'homogénat est ensuite ultracentrifugé (105 000 g × 90 mn) à 0 °C. Des aliquotes du surnageant ainsi obtenu, sont incubées à 0 °C ou à 25 °C pendant un temps (t) avec une concentration constante (T) d'estradiol tritié en présence de concentrations croissantes (0–1000.10 $^{9}$M) soit d'estradiol froid, soit du produit froid à tester. La concentration d'estradiol tritié lié (B) est ensuite mesurée dans chaque incubat par la technique d'adsorption au charbon-dextran.

Calcul de l'affinité relative de liaison:

Le calcul de l'affinité relative de liaison (ARL) est identique pour tous les récepteurs.

On trace les 2 courbes suivantes: le pourcentage de l'hormone tritiée liée $\frac{B}{T}$ en fonction du logarithme de la concentration de l'hormone de référence froide et $\frac{B}{T}$ en fonction du logarithme de la concentration du produit froid testé.

On détermine la droite d'équation $I_{50} = (\frac{B}{T} max + \frac{B}{T} min)/2$. $\frac{B}{T}$ max = Pourcentage de l'hormone tritiée liée pour une incubation de cette hormone tritiée à la concentration (T). $\frac{B}{T}$ min = Pourcentage de l'hormone tritiée liée pour une incubation de cette hormone tritiée à la concentration (T) en présence d'un grand excès d'hormone froide (2500.10 $^{9}$M).

Les intersections de la droite $I_{50}$ et des courbes permettent d'évaluer les concentrations de l'hormone de référence froide (CH) et du produit froid testé (CX) qui inhibent de 50% la liaison de l'hormone tritiée sur le récepteur.

L'affinité relative de liaison (ARL) du produit testé est déterminée par l'équation $ARL = 100 \frac{(CH)}{(CX)}$

Les résultats obtenus sont les suivants:

| Temps d'incubation à 0 °C<br>Produits des exemples | Minéralo-corticoïde | | Androgène | | Progéstogène | | Glucocorticoïde | | Estrogène 5H | |
|---|---|---|---|---|---|---|---|---|---|---|
| | 1 H | 24 H | 1/2 H | 24 H | 2 H | 24 H | 4 H | 24 H | 2 H | (25 °C) |
| 1 | < 0,1 | < 0,1 | – | – | 4 | 4 | 97 | 38 | < 0,1 | < 0,1 |
| 2 (17 OH) | 0,2 | < 0,1 | * | * | 39 | 130 | 153 | 222 | 5 | < 0,1 |
| 2 (17 OAö) | < 0,1 | < 0,1 | < 0,1 | < 0,1 | 5 | 52 | 58 | 100 | < 0,1 | 0,3 |
| 5 (2α) | < 0,1 | < 0,1 | 0,5 | 0,4 | 40 | 39 | 214 | 255 | < 0,1 | < 0,1 |
| 5 (2β) | < 0,1 | < 0,1 | 0,7 | 0,5 | 44 | 44 | 224 | 299 | < 0,1 | < 0,1 |
| 9 | < 0,1 | < 0,1 | < 0,1 | < 0,1 | < 0,1 | < 0,1 | 14 | 7 | < 0,1 | < 0,1 |
| 10 | 1,5 | < 0,1 | – | – | 27 | 62 | 83 | 115 | < 0,1 | < 0,1 |
| 13 | – | – | 5 | 6 | – | – | 3,9 | – | 78 | 239 |
| 14 | – | – | 10,6 | 0,5 | – | – | 0,4 | – | 4,1 | 1,4 |
| 15 | – | – | 1,3 | 0,2 | – | – | 2 | – | 0,1 | 0,1 |

*: Le produit présente une affinité pour le récepteur.

Conclusion:

Les produits étudiés, particulièrement les produits des exemples 2 et 5 présentent une affinité très marquée pour les récepteurs glucocorticoïde et progestogène, ainsi qu'une affinité modérée pour le récepteur androgène.

Des résultats obtenus on peut conclure que les produits peuvent présenter des activités agonistes ou antagonistes des glucocorticoïdes, des progestogènes et des androgènes. On peut conclure en outre que les produits des exemples 13 et 14 présentent des activités agonistes ou antagonistes des estrogènes.

II) Activité antiglucocorticoïde:

La technique utilisée découle de la méthode décrite par Dausse et Coll dans Molecular Pharmacology 13, 948–955 (1977) («the relationship beetween glucocorticoïd structure and effects upon Thymocytes»), pour des thymocytes de souris.

Des thymocytes de rats surrénalectomisés sont incubés à 37 °C pendant 3 heures, dans un milieu nutritif renfermant 5.10 $^{8}$M de dexaméthasone en présence ou non d'un produit à étudier à différentes concentrations. On ajoute l'uridine tritiée, et poursuit l'incubation pendant une heure. On refroidit les incubats, les traite avec une solution d'acide trichloroacétique à 5%, les filtre sur papier Whatman GF/A, les lave trois fois à l'aide d'une solution d'acide trichloroacétique à 5%. On détermine la radioactivité retenue par le filtre.

Les glucocorticoïdes et en particulier la dexaméthasone provoquent une diminution de l'incorporation d'uridine tritiée. Les produits des exemples 1, 2, 5 et 10 s'opposent à cet effet.

| Produit de l'exemple: | 5.10 $^{8}$ Dexaméthasone + produit à tester à la concentration de: | % d'inhibition de l'effet de la Dexaméthasone |
|---|---|---|
| 1 | 10 $^{8}$M | 8 |
| | 10 $^{7}$M | 18 |
| | 10 $^{6}$M | * |
| 2 (17 OH) | 10 $^{8}$M | 41 |
| | 10 $^{7}$M | 91 |
| | 10 $^{6}$M | * |
| 2 (17 OAc) | 10 $^{8}$M | 24 |
| | 10 $^{7}$M | 76 |
| | 10 $^{6}$M | * |
| 3 | 10 $^{8}$M | 0 |
| | 10 $^{7}$M | 0 |
| | 10 $^{6}$M | 60 |
| 4 | 10 $^{8}$M | 0 |
| | 10 $^{7}$M | 0 |
| | $10^{-6}$M | 51 |
| 5 (2α) | 10 $^{8}$M | 19 |
| | 10 $^{7}$M | 57 |
| | 10 $^{6}$M | 100 |
| 5 (2β) | 10 $^{8}$M | 10 |
| | 10 $^{7}$M | 57 |
| | 10 $^{6}$M | * |
| 7 | 10 $^{8}$M | 0 |
| | 10 $^{7}$M | 27 |
| | 10 $^{6}$M | * |
| 9 | 10 $^{8}$M | 3 |
| | 10 $^{7}$M | 1 |
| | 10 $^{6}$M | 2 |
| 10 | 10 $^{8}$M | 0 |
| | 10 $^{7}$M | 23 |
| | 10 $^{6}$M | * |

| Produit de l'exemple: | $5.10^{-8}$ Dexaméthasone + produit à tester à la concentration de: | % d'inhibition de l'effet de la Dexaméthasone |
|---|---|---|
| 6 | $10^{-8}$M | 0 |
| | $10^{-7}$M | 0 |
| | $10^{-6}$M | 68 |
| 6 (17α propényle) | $10^{-8}$M | 0 |
| | $10^{-7}$M | 0 |
| | $10^{-6}$M | 56 |

*: A la dose de $10^{-6}$M l'inhibition de l'effet de la déxaméthasone a été totale.

Il a par ailleurs été constaté qu'utilisés seuls les produits testés ne provoquent aucun effet du type glucocorticoïde aux doses provoquant l'effet antagoniste.

Conclusion:

Les produits étudiés présentent une activité anti-glucocorticoïde très marquée tout en étant dépourvus d'activité glucocorticoïde.

Compositions pharmaceutiques:

On a préparé des comprimés répondant à la formule suivante:

- Produit de l'exemple 5 (2β) 50 mg
  Excipient (talc, amidon, stéarate de magnésium) q.s. pour un comprimé terminé à 120 mg

**Revendications pour les Etats contractants BE, CH, DE, FR, GB, IT, LI, LU, NL, SE**

1. Les produits de formule générale $I_D$:

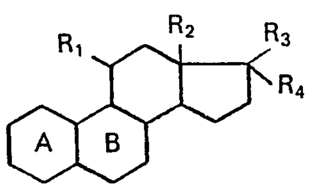

$(I_D)$

dans laquelle $R_1$ représente un radical organique renfermant de 1 à 18 atomes de carbone et éventuellement un ou plusieurs hétéroatomes, l'atome immédiatement adjacent au carbone en 11 étant un atome de carbone, $R_2$ représente un radical hydrocarboné renfermant de 1 à 8 atomes de carbone, les cycles A et B ayant l'une des structures suivantes:

a) – Soit A et B représentent le groupement:

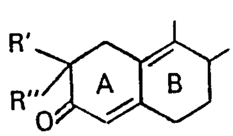

dans lequel R' et R'' identiques ou différents représentent un atome d'hydrogène, un radical nitrile ou un radical alkyle ayant de 1 à 4 atomes de carbone étant entendu que l'un au moins des radicaux R' ou R'' ne représente pas un atome d'hydrogène;

b) – Soit A et B représentent le groupement:

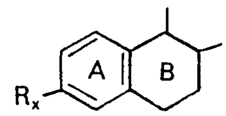

Rx représentant un atome d'hydrogène ou un groupement –ORe, dans lequel Re représente un atome d'hydrogène, un radical alkyle ayant de 1 à 6 atomes de carbone éventuellement substitué ou un radical acyle;

c) – Soit A et B représentent le groupement:

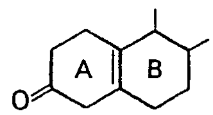

étant entendu que lorsque A et B représentent le groupement

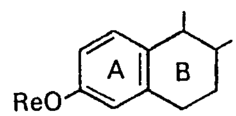

le radical $R_1$ contient au moins un atome d'azote, de phosphore ou de silicium, et que lorsque A et B représentent le groupement

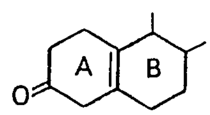

le radical $R_1$ ne représente pas un radical alkyle saturé linéaire, ainsi que les sels d'addition des produits de formule I avec les acides, et $R_3$ et $R_4$ forment ensemble le radical:

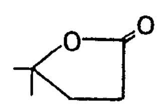

2. Les produits de formule $I_D$ telle que définie à la revendication 1 dans laquelle $R_1$ représente un radical aryle ou aralkyle portant une fonction amine:

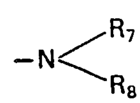

dans laquelle $R_7$ et $R_8$ représentent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisi dans le groupe constitué par l'oxygène, l'azote, le soufre

et le silicium dont au moins un atome d'azote, ou substitué par un hétérocycle comportant au moins un atome d'azote.

3. Les produits de formule $I_D$ telle que définie à l'une quelconque des revendications 1 et 2 dans laquelle $R_1$ représente le radical:

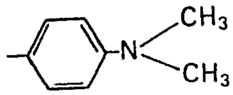

$R_2$ représente le radical $CH_3$ et 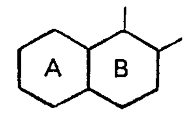

représente les radicaux:

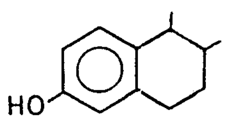 ; 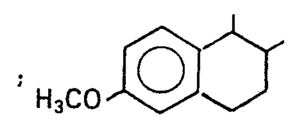 ;

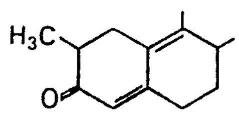 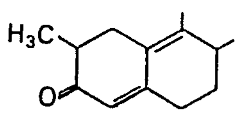

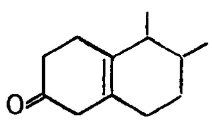 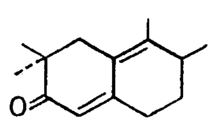

4. Procédé de préparation des produits de formule générale $I_D$ telle que définie à la revendication 1 caractérisé en ce que:

a) pour préparer les produits de formule $I_{DA}$:

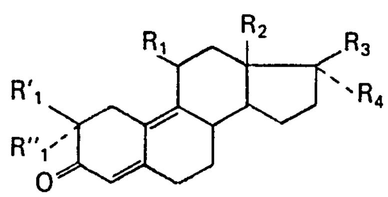

($I_{DA}$)

dans laquelle $R_1$, $R_2$, $R_3$ et $R_4$ conservent la même signification qu'à la revendication 1 et $R_1'$ et $R_1''$ sont tels que, ou bien $R_1'$ et $R_1''$ représentent chacun un radical alkyle ou bien l'un représente un atome d'hydrogène et l'autre représente un radical alkyle ou bien $R_1'$ et $R_1''$ représentent chacun un radical nitrile ou bien l'un représente un radical alkyle et l'autre un radical nitrile, on soumet un produit de formule $II_D$:

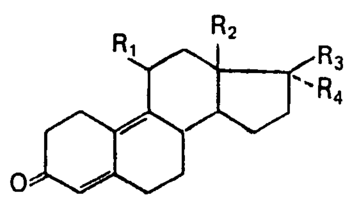

($II_D$)

dans laquelle $R_1$, $R_2$, $R_3$ et $R_4$ conservent la même signification qu'à la revendication 1, d'abord, éventuellement à l'action d'un réactif de protection des groupements fonctionnels puis à l'action d'une base forte et ou bien d'un halogénure d'alkyle ou bien du cyanure de tosyle, ou bien d'abord à l'action d'un halogénure d'alkyle puis du cyanure de tosyle, puis élimine si nécessaire le groupement protecteur;

b) 1) pour préparer les produits de formule $I_{DB}$:

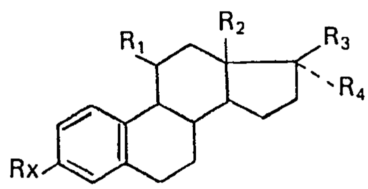

($I_{DB}$)

répondant à la formule $I_{DB1}$:

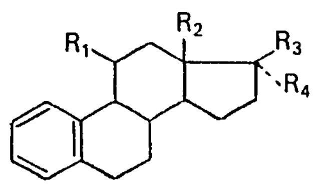

($I_{DB1}$)

dans laquelle $R_1$, $R_2$, $R_3$ et $R_4$ conservent la même signification qu'à la revendication 1, on soumet un produit de formule $II_D$ à l'action d'un agent réducteur, pour obtenir un produit de formule $II_{D1}$:

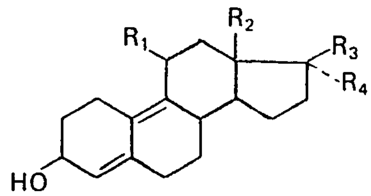

($II_{D1}$)

dans laquelle $R_1$, $R_2$, $R_3$ et $R_4$ conservent la même signification que ci-dessus, que l'on soumet à l'action d'un agent d'aromatisation acide;

b) 2) pour préparer les produits de formule $I_{DB}$ répondant à la formule $I_{DB2}$:

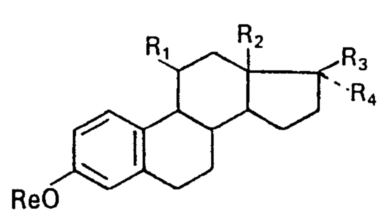

($I_{DB2}$)

dans laquelle $R_1$, $R_2$, Re, $R_3$ et $R_4$ conservent la même signification que ci-dessus, on soumet un produit de formule $II_D$ à l'action d'un agent d'aromatisation puis de saponification, puis éventuellement à un réactif d'alkylation ou d'acylation;

c) pour préparer les produits de formule $I_{DC}$:

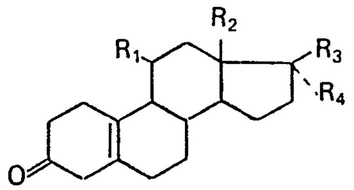

($I_{DC}$)

dans laquelle $R_1$, $R_2$, $R_3$ et $R_4$ conservent la même signification qu'à la revendication 1, on soumet un produit de formule $II_D$ à l'action d'un agent réducteur;

d) pour préparer les produits de formule $I'_{DA}$

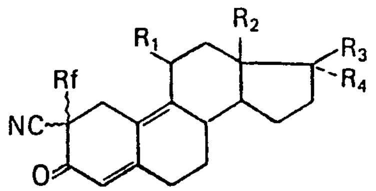

($I'_{DA}$)

dans laquelle Rf représente un atome d'hydrogène ou un radical alkyle et $R_1$, $R_2$, $R_3$ et $R_4$ conservent la même signification qu'à la revendication 1, on fait agir une base sur les produits de formule $I_{DE}$ pour obtenir les produits de formule $I'_{DA}$ dans laquelle Rf représente un atome d'hydrogène et si désiré l'on soumet les produits ainsi obtenus à l'action d'une base forte et d'un halogénure d'alkyle pour obtenir les produits de formule $I'_{DA}$ dans laquelle Rf représente un radical alkyle.

5. Les produits de formule $II_D$:

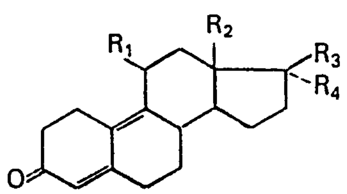

($II_D$)

dans laquelle $R_1$, $R_2$, $R_3$ et $R_4$ conservent la même signification qu'à la revendication 1.

6. Produit de formule $II_D$ telle que définie à la revendication 5 dans laquelle $R_2$ représente un radical méthyle et $R_1$ représente un radical:

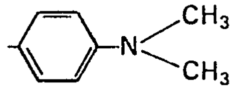

7. A titre de médicaments, les produits de formule I telle que définie à l'une des revendications 1 à 3.

8. Les compositions pharmaceutiques renfermant comme principe actif un au moins des médicaments selon la revendication 7.

**Revendicaitons pour l'état contractant AT**

1. Procédé de préparation des produits de formule générale $I_D$:

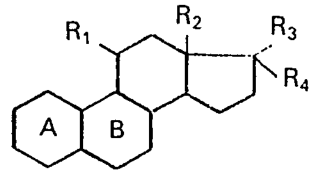

($I_D$)

dans laquelle $R_1$ représente un radical organique renfermant de 1 à 18 atomes de carbone et éventuellement un ou plusieurs hétéroatomes, l'atome immédiatement adjacent au carbone en 11 étant un atome de carbone, $R_2$ représente un radical hydrocarboné renfermant de 1 à 8 atomes de carbone, les cycles A et B ayant l'une des structures suivantes:

a) – Soit A et B représentent le groupement:

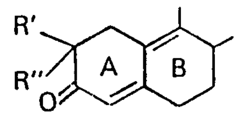

dans lequel R' et R'' identiques ou différents représentent un atome d'hydrogène, un radical nitrile ou un radical alkyle ayant de 1 à 4 atomes de carbone étant entendu que l'un au moins des radicaux R' ou R'' ne représente pas un atome d'hydrogène;

b) – Soit A et B représentent le groupement:

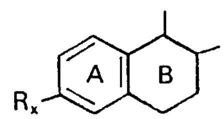

Rx représentant un atome d'hydrogène ou un groupement –ORe, dans lequel Re représente un atome d'hydrogène, un radical alkyle ayant de 1 à 6 atomes de carbone éventuellement substitué ou un radical acyle;

c) – Soit A et B représentent le groupement:

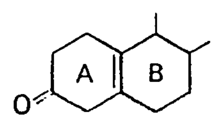

étant entendu que lorsque A et B représentent le groupement

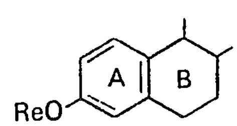

le radical $R_1$ contient au moins un atome d'azote, de phosphore ou de silicium, et que lorsque A et B représentent le groupement

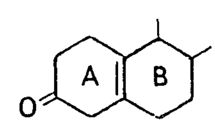

le radical $R_1$ ne représente pas un radical alkyle saturé linéaire, ainsi que les sels d'addition des produits de formule I avec les acides, et $R_3$ et $R_4$ forment ensemble le radical:

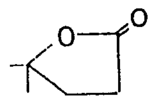

caractérisé en ce que:

a) pour préparer les produits de formule $I_{DA}$:

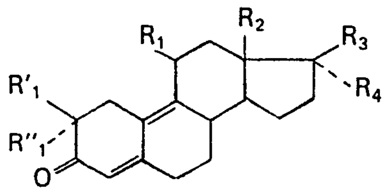

($I_{DA}$)

dans laquelle $R_1$, $R_2$, $R_3$ et $R_4$ conservent la même signification que ci-dessus et $R_1'$ et $R_1''$ sont tels que, ou bien $R_1'$ et $R_1''$ représentent chacun un radical alkyle ou bien l'un représente un atome d'hydrogène et l'autre représente un radical alkyle ou bien $R_1'$ et $R_1''$ représentent chacun un radical nitrile ou bien l'un représente un radical alkyle et l'autre un radical nitrile, on soumet un produit de formule $II_D$:

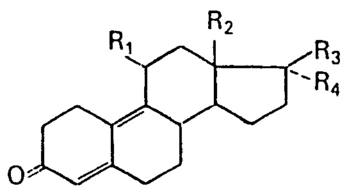

dans laquelle $R_1$, $R_2$, $R_3$ et $R_4$ conservent la même signification que ci-dessus d'abord, éventuellement à l'action d'un réactif de protection des groupements fonctionnels puis à l'action d'une base forte et ou bien d'un halogénure d'alkyle ou bien du cyanure de tosyle, ou bien d'abord à l'action d'un halogénure d'alkyle puis du cyanure de tosyle, puis élimine si nécessaire le groupement protecteur;

b) 1) pour préparer les produits de formule $I_{DB}$:

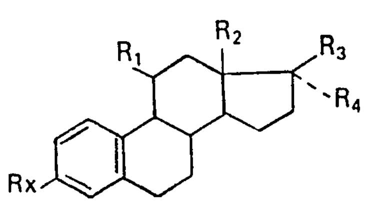

($I_{DB}$)

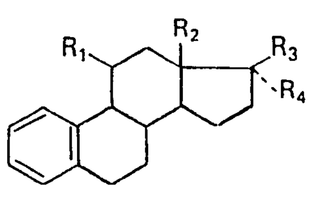

($I_{DB1}$)

dans laquelle $R_1$, $R_2$, $R_3$ et $R_4$ conservent la même signification que ci-dessus, on soumet un produit de formule $II_D$ à l'action d'un agent réducteur, pour obtenir un produit de formule $II_{D1}$:

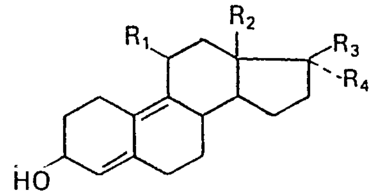

($II_{D1}$)

dans laquelle $R_1$, $R_2$, $R_3$ et $R_4$ conservent la même signification que ci-dessus, que l'on soumet à l'action d'un agent d'aromatisation acide;

b) 2) pour préparer les produits de formule $I_{DB}$ répondant à la formule $I_{DB2}$:

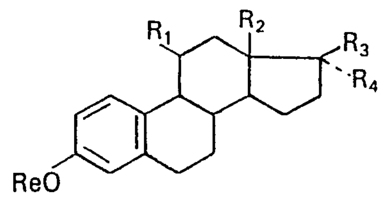

($I_{DB2}$)

dans laquelle $R_1$, $R_2$, Re, $R_3$ et $R_4$ conservent la même signification que ci-dessus, on soumet un produit de formule $II_D$ à l'action d'un agent d'aromatisation puis de saponification, puis éventuellement à un réactif d'alkylation ou d'acylation;

c) pour préparer les produits de formule $I_{DC}$:

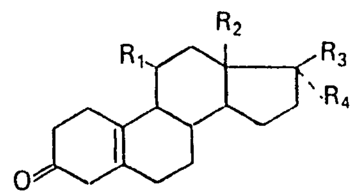

($I_{DC}$)

dans laquelle $R_1$, $R_2$, $R_3$ et $R_4$ conservent la même signification que ci-dessus, on soumet un produit de formule $II_D$ à l'action d'un agent réducteur;

d) pour préparer les produits de formule $I'_{DA}$

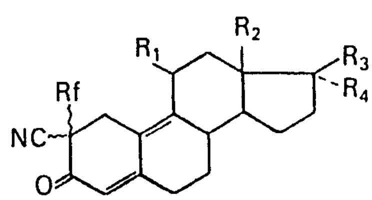

($I'_{DA}$)

dans laquelle Rf représente un atome d'hydrogène ou un radical alkyle et $R_1$, $R_2$, $R_3$ et $R_4$ conservent la même signification qu'à la revendication 1, on fait agir une base sur les produits de formule $I_{DE}$ pour obtenir les produits de formule $I'_{DA}$ dans laquelle Rf représente un atome d'hydrogène et si désiré l'on soumet les produits ainsi obtenus à l'action d'une base forte et d'un halogénure d'alkyle pour obtenir les produits de formule $I'_{DA}$ dans laquelle Rf représente un radical alkyle.

2. Procédé selon la revendication 1 caractérisé en ce que l'on prépare les produits de formule $I_D$ dans laquelle $R_1$ représente un radical aryle ou aralkyle portant une fonction amine:

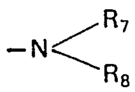

dans laquelle $R_7$ et $R_8$ représentent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisi dans le groupe constitué par l'oxygène, l'azote, le soufre et le silicium dont au moins un atome d'azote, ou substitué par un hétérocycle comportant au moins un atome d'azote.

3. Procédé selon l'une des revendications 1 ou 2 caractérisé en ce que l'on prépare les produits de formule $I_D$ dans laquelle $R_1$ représente le radical:

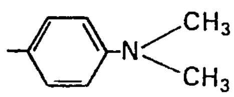

$R_2$ représente le radical $CH_3$ et 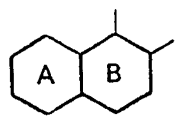

représente les radicaux:

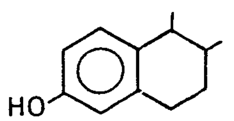 ; 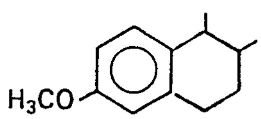 ;

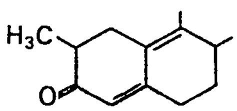 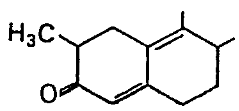

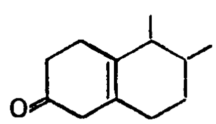 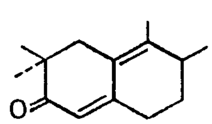

**Patentansprüche für die Vertragsstaaten: BE, CH, DE, FR, GB, IT, LI, LU, NL, SE**

1. Die Produkte der allgemeinen Formel ($I_D$)

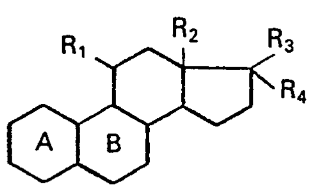

($I_D$)

worin $R_1$ einen organischen Rest mit 1 bis 18 Kohlenstoffatomen und gegebenenfalls einem oder mehreren Heteroatomen bedeutet, wobei das dem Kohlenstoffatom in 11-Stellung unmittelbar benachbarte Atom ein Kohlenstoffatom ist, $R_2$ für einen Kohlenwasserstoffrest mit 1 bis 8 Kohlenstoffatomen steht und die Ringe A und B eine der folgenden Strukturen besitzen:

(a) – entweder A und B die Gruppe

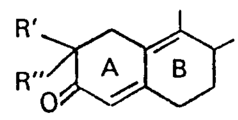

wiedergeben, worin R' und R", die gleich oder voneinander verschieden sind, ein Wasserstoffatom, einen Nitrilrest oder einen Alkylrest mit 1 bis 4 Kohlenstoffatomen bedeuten, mit der Massgabe, dass zumindest einer der Reste R' oder R" kein Wasserstoffatom darstellt;

(b) – oder A und B für eine Gruppe

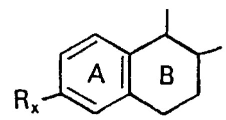

stehen, worin Rx ein Wasserstoffatom oder eine Gruppe –ORe wiedergibt, in der Re ein Wasserstoffatom, einen gegebenenfalls substituierten Alkylrest mit 1 bis 6 Kohlenstoffatomen oder einen Acylrest wiedergibt;

(c) – oder A und B für die Gruppe

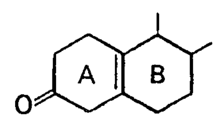

stehen, mit der Massgabe, dass, wenn A und B die Gruppe

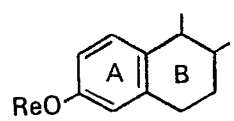

bedeuten, der Rest $R_1$ zumindest ein Stickstoff-, Phosphor- oder Siliciumatom enthält, und wenn A und B die Gruppe

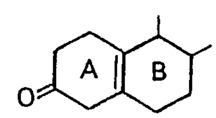

wiedergeben, der Rest $R_1$ keinen linearen, gesättigten Alkylrest wiedergibt, sowie die Additionssalze der Produkte der Formel I mit Säuren, und

$R_3$ und $R_4$ gemeinsam den Rest 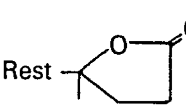 bilden.

2. Die Produkte der Formel $I_D$ gemäss Anspruch 1, worin $R_1$ einen Aryl- oder Aralkylrest mit einer Aminfunktion 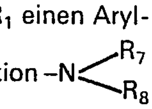 bedeutet, worin $R_7$ und $R_8$ einen Alkylrest mit 1 bis 8 Kohlenstoffatomen oder einen primären, sekundären oder tertiären Alkylrest mit 1 bis 8 Kohlenstoffatomen wiedergeben, der ein oder mehrere Heteroatome, ausgewählt unter Sauerstoff, Stickstoff, Schwefel und

Silicium, und unter diesen zumindest ein Stickstoffatom aufweist oder substituiert ist durch einen Heterocyclus mit zumindest einem Stickstoffatom.

3. Die Produkte der Formel $I_D$ gemäss einem der Ansprüche 1 und 2, worin

$R_1$ den Rest 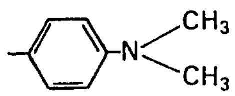 wiedergibt,

$R_2$ den Methylrest bedeutet und 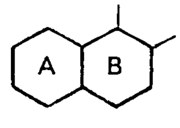

für die folgenden Reste steht:

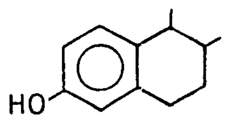 ; 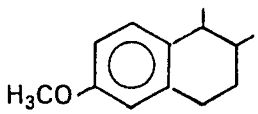 ;

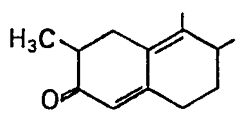

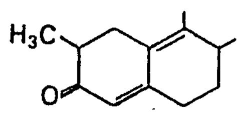

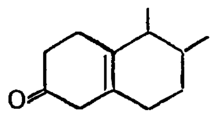

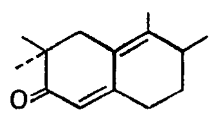

4. Verfahren zur Herstellung der Produkte der allgemeinen Formel $I_D$ gemäss Anspruch 1, dadurch gekennzeichnet, dass man

(a) zur Herstellung der Produkte der Formel $I_{DA}$

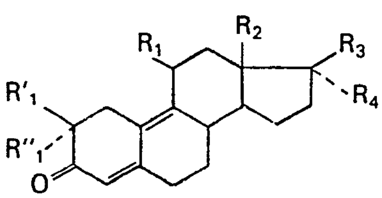

($I_{DA}$)

worin $R_1$, $R_2$, $R_3$ und $R_4$ die in Anspruch 1 gegebene Bedeutung haben und $R_1'$ und $R_1''$ derart sind, dass entweder $R_1'$ und $R_1''$ jeweils einen Alkylrest bedeuten oder einer dieser Reste für ein Wasserstoffatom steht und der andere für einen Alkylrest oder $R_1'$ und $R_1''$ jeweils eine Nitrilgruppe bedeuten oder einer dieser Reste einen Alkylrest und der andere einen Nitrilrest darstellt, ein Produkt der Formel $II_D$

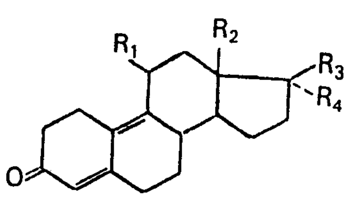

($II_D$)

worin $R_1$, $R_2$, $R_3$ und $R_4$ wie in Anspruch 1 definiert sind, zunächst gegebenenfalls der Einwirkung eines Reagens zum Schutz der funktionellen Gruppen, danach derjenigen einer starken Base und entweder eines Alkylhalogenids oder eines Tosylcyanids unterzieht oder zunächst der Einwirkung eines Alkylhalogenids und dann derjenigen eines Tosylcyanids unterzieht und hiernach erforderlichenfalls die Schutzgruppe entfernt;

(b) (1) zur Herstellung der Produkte der Formel $I_{DB}$

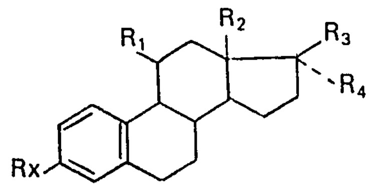

($I_{DB}$)

entsprechend der Formel $I_{DB1}$

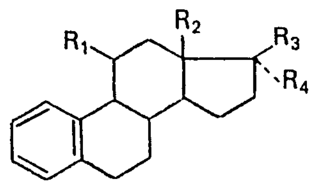

($I_{DB1}$)

worin $R_1$, $R_2$, $R_3$ und $R_4$ wie in Anspruch 1 definiert sind, ein Produkt der Formel $II_D$ der Einwirkung eines Reduktionsmittels unterzieht, um zu einem Produkt der Formel $II_{D1}$

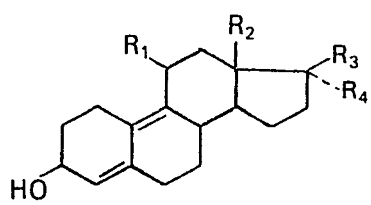

($II_{D1}$)

zu gelangen, worin $R_1$, $R_2$, $R_3$ und $R_4$ wie vorstehend definiert sind, das man der Einwirkung eines Mittels für die saure Aromatisierung unterzieht;

(b) (2) zur Herstellung der Produkte der Formel $I_{DB}$ entsprechend der Formel $I_{DB2}$

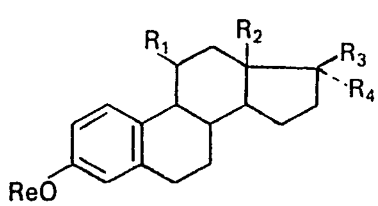

($I_{DB2}$)

worin $R_1$, $R_2$, Re, $R_3$ und $R_4$ wie vorstehend definiert sind, ein Produkt der Formel $II_D$ der Einwirkung eines Aromatisierungsmittels und danach derjenigen eines Verseifungsmittels und hiernach gegebenenfalls eines Alkylierungs- oder Acylierungsreagens unterzieht;

(c) zur Herstellung der Produkte der Formel $I_{DC}$

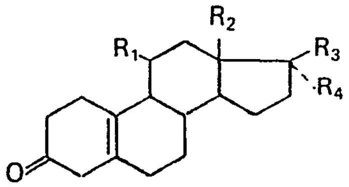

($I_{DC}$)

worin $R_1$, $R_2$, $R_3$ und $R_4$ wie in Anspruch 1 definiert sind, ein Produkt der Formel $II_D$ der Einwirkung eines Reduktionsmittels unterzieht;

(d) zur Herstellung der Produkte der Formel $I'_{DA}$

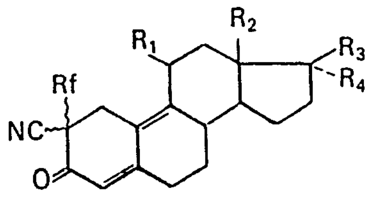

($I'_{DA}$)

worin Rf für ein Wasserstoffatom oder einen Alkylrest steht und $R_1$, $R_2$, $R_3$ und $R_4$ die in Anspruch 1 angegebene Bedeutung haben, eine Base mit den Produkten der Formel $I_{DE}$ umsetzt, um zu den Produkten der Formel $I'_{DA}$ zu gelangen, worin Rf für ein Wasserstoffatom steht, und die so erhaltenen Produkte gewünschtenfalls der Einwirkung einer starken Base und eines Alkylhalogenids unterzieht, um zu den Produkten der Formel $I'_{DA}$ zu gelangen, worin Rf für einen Alkylrest steht.

5. Die Produkte der Formel $II_D$

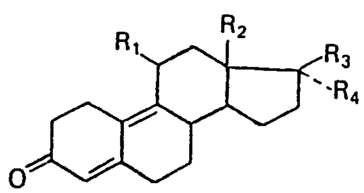

($II_D$)

worin $R_1$, $R_2$, $R_3$ und $R_4$ wie in Anspruch 1 definiert sind.

6. Produkt der Formel $II_D$ gemäss Anspruch 5, worin $R_2$ für einen Methylrest steht und $R_1$ den

Rest 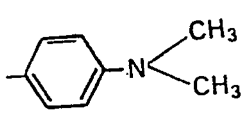 wiedergibt.

7. Als Arzneimittel die Produkte der Formel I gemäss einem der Ansprüche 1 bis 3.

8. Pharmazeutische Zusammensetzungen, enthaltend als Wirkstoff zumindest eines der Arzneimittel gemäss Anspruch 7.

**Patentansprüche für den Vertragsstaat: AT**

1. Verfahren zur Herstellung der Produkte der allgemeinen Formel $I_D$

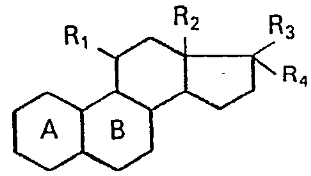

($I_D$)

worin $R_1$ einen organischen Rest mit 1 bis 18 Kohlenstoffatomen und gegebenenfalls einem oder mehreren Heteroatomen bedeutet, wobei das dem Kohlenstoffatom in 11-Stellung unmittelbar benachbarte Atom ein Kohlenstoffatom ist, $R_2$ für einen Kohlenwasserstoffrest mit 1 bis 8 Kohlenstoffatomen steht und die Ringe A und B eine der folgenden Strukturen besitzen:

(a) – entweder A und B die Gruppe

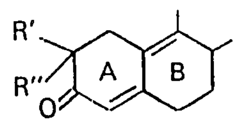

wiedergeben, worin R' und R'', die gleich oder voneinander verschieden sind, ein Wasserstoffatom, einen Nitrilrest oder einen Alkylrest mit 1 bis 4 Kohlenstoffatomen bedeuten, mit der Massgabe, dass zumindest einer der Reste R' oder R'' kein Wasserstoffatom darstellt;

(b) – oder A und B für eine Gruppe

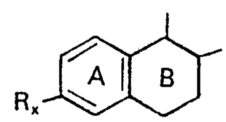

stehen, worin Rx ein Wasserstoffatom oder eine Gruppe –ORe wiedergibt, in der Re ein Wasserstoffatom, einen gegebenenfalls substituierten Alkylrest mit 1 bis 6 Kohlenstoffatomen oder einen Acylrest wiedergibt;

(c) – oder A und B für die Gruppe

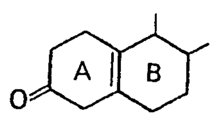

stehen, mit der Massgabe, dass, wenn A und B die Gruppe

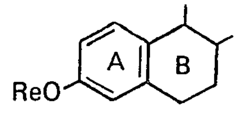

bedeuten, der Rest $R_1$ zumindest ein Stickstoff-, Phosphor- oder Siliciumatom enthält, und wenn A und B die Gruppe

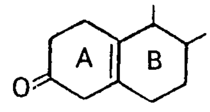

wiedergeben, der Rest $R_1$ keinen linearen, gesät-

tigten Alkylrest wiedergibt, sowie die Additionssalze der Produkte der Formel I mit Säuren, und

$R_3$ und $R_4$ gemeinsam den Rest 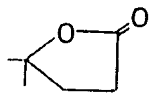

bilden, dadurch gekennzeichnet, dass man

(a) zur Herstellung der Produkte der Formel $I_{DA}$

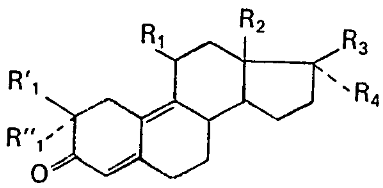

($I_{DA}$)

worin $R_1$, $R_2$, $R_3$ und $R_4$ die vorstehend angegebene Bedeutung haben und $R'_1$ und $R''_1$ derart sind, dass entweder $R'_1$ und $R''_1$ jeweils einen Alkylrest bedeuten oder einer dieser Reste für ein Wasserstoffatom steht und der andere für einen Alkylrest oder $R'_1$ und $R''_1$ jeweils eine Nitrilgruppe bedeuten oder einer dieser Reste einen Alkylrest und der andere einen Nitrilrest darstellt, ein Produkt der Formel $II_D$

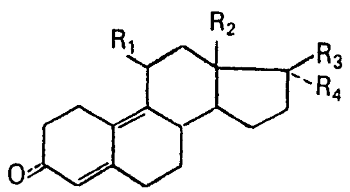

($II_D$)

worin $R_1$, $R_2$, $R_3$ und $R_4$ die vorstehend angegebene Bedeutung haben, zunächst gegebenenfalls der Einwirkung eines Reagens zum Schutz der funktionellen Gruppen, danach derjenigen einer starken Base und entweder eines Alkylhalogenids oder eines Tosylcyanids unterzieht oder zunächst der Einwirkung eines Alkylhalogenids und dann derjenigen eines Tosylcyanids unterzieht und hiernach erforderlichenfalls die Schutzgruppe entfernt;

(b) (1) zur Herstellung der Produkte der Formel $I_{DB}$

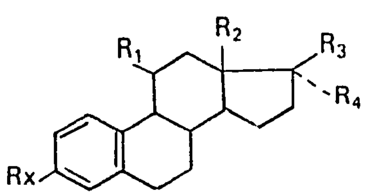

($I_{DB}$)

entsprechend der Formel $I_{DB1}$

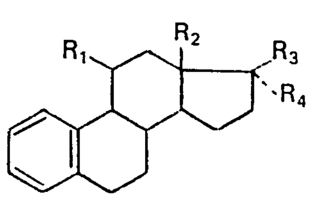

($I_{DB1}$)

worin $R_1$, $R_2$, $R_3$ und $R_4$ wie vorstehend definiert sind, ein Produkt der Formel $II_D$ der Einwirkung eines Reduktionsmittels unterzieht, um zu einem Produkt der Formel $II_{D1}$

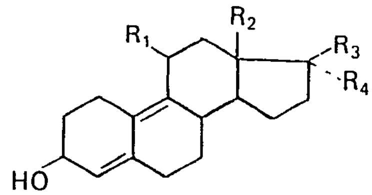

($II_{D1}$)

zu gelangen, worin $R_1$, $R_2$, $R_3$ und $R_4$ wie vorstehend definiert sind, das man der Einwirkung eines Mittels für die saure Aromatisierung unterzieht;

(b) (2) zur Herstellung der Produkte der Formel $I_{DB}$ entsprechend der Formel $I_{DB2}$

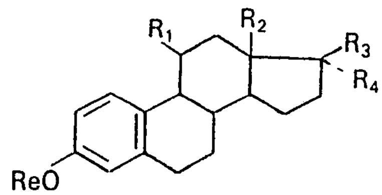

($I_{DB2}$)

worin $R_1$, $R_2$, Re, $R_3$ und $R_4$ wie vorstehend definiert sind, ein Produkt der Formel $II_D$ der Einwirkung eines Aromatisierungsmittels und danach derjenigen eines Verseifungsmittels und hiernach gegebenenfalls eines Alkylierungs- oder Acylierungsreagens unterzieht;

(c) zur Herstellung der Produkte der Formel $I_{DC}$

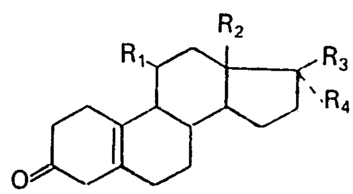

($I_{DC}$)

worin $R_1$, $R_2$, $R_3$ und $R_4$ wie vorstehend definiert sind, ein Produkt der Formel $II_D$ der Einwirkung eines Reduktionsmittels unterzieht;

(d) zur Herstellung der Produkte der Formel $I'_{DA}$

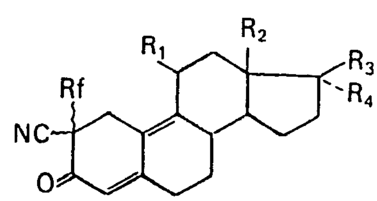

($I'_{DA}$)

worin Rf für ein Wasserstoffatom oder einen Alkylrest steht und $R_1$, $R_2$, $R_3$ und $R_4$ wie vorstehend definiert sind, eine Base mit den Produkten der Formel $I_{DE}$ umsetzt, um zu den Produkten der Formel $I'_{DA}$ zu gelangen, worin Rf für ein Wasserstoffatom steht, und die so erhaltenen Produkte gewünschtenfalls der Einwirkung einer starken Base und eines Alkylhalogenids unterzieht, um zu den Produkten der Formel $I'_{DA}$ zu gelangen, worin Rf für einen Alkylrest steht.

2. Verfahren gemäss Anspruch 1, dadurch gekennzeichnet, dass man die Produkte der Formel $I_D$ herstellt, worin $R_1$ für einen Aryl- oder Aralkylrest mit einer Aminfunktion 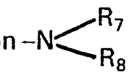 bedeutet, worin $R_7$ und $R_8$ einen Alkylrest mit 1 bis 8 Kohlenstoffatomen oder einen primären, sekundären oder tertiären Alkylrest mit 1 bis 8 Kohlenstoffatomen wiedergeben, der ein oder mehrere Heteroatome, ausgewählt unter Sauerstoff, Stickstoff, Schwefel und Silicium, und unter diesen zumindest ein Stickstoffatom aufweist oder substituiert ist durch einen Heterocyclus mit zumindest einem Stickstoffatom.

3. Verfahren gemäss einem der Ansprüche 1 oder 2, dadurch gekennzeichnet, dass man die Produkte der Formel $I_D$ herstellt, worin $R_1$ den

Rest: 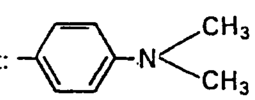 wiedergibt, $R_2$ für einen Rest

$CH_3$ steht und 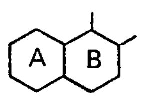

die folgenden Reste

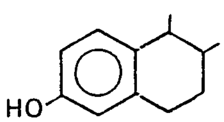 ; 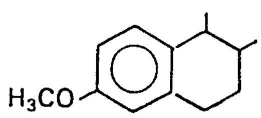 ;

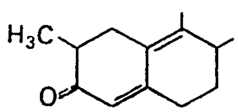 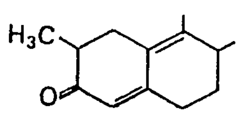

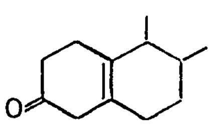 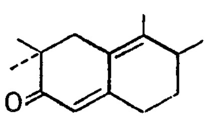

wiedergibt.

**Claims for the contracting states: BE, CH, DE, FR, GB, IT, LI, LU, NL, SE**

1. Products of the general formula $I_D$:

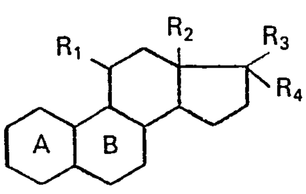

($I_D$)

in which $R_1$ represents an organic radical containing from 1 to 18 carbon atoms and optionally one or more heteroatoms, the atom immediately next to the carbon in position 11 being a carbon atom, $R_2$ represents a hydrocarbon radical containing from 1 to 8 carbon atoms, the rings A and B having the following structures:

a) – Either A and B represent the group:

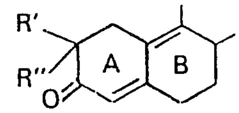

in which R' and R'', identical or different, represent a hydrogen atom, a nitrile radical or an alkyl radical having from 1 to 4 carbon atoms, it being understood that at least one of the radicals R' or R'' does not represent a hydrogen atom;

b) – Or A and B represent the group:

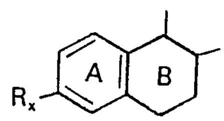

Rx representing a hydrogen atom or a -ORe group, in which Re represents a hydrogen atom, an alkyl radical containing from 1 to 6 carbon atoms optionally substituted or an acyl radical;

c) – Or A and B represent the group:

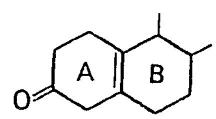

it bein understood that when A and B represent the group

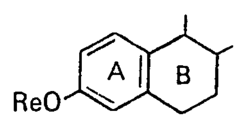

the radical $R_1$ contains at least one nitrogen, phosphorus or silicon atom, and that when A and B represent the group

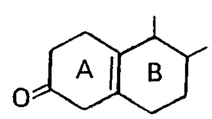

the radical $R_1$ does not represent a saturated linear alkyl radical, as well as the addition salts of the products of formula I with acids, and $R_3$ and $R_4$ together form the radical:

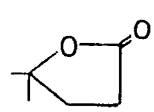

2. Products of formula $I_D$ as defined in claim 1 in which $R_1$ represents an aryl or aralkyl radical bearing an amine function:

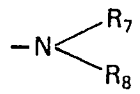

in which $R_7$ and $R_8$ represent an alkyl radical containing from 1 to 8 carbon atoms or a primary, secondary or tertiary alkyl radical containing from 1 to 8 carbon atoms, including one or more heteroatoms selected from the group constituted by oxygen, nitrogen, sulphur and silicon, of which there is at least one nitrogen atom, or substituted by a heterocycle containing at least one nitrogen atom.

3. Products of the formula $I_D$ as defined in either one of claims 1 and 2 in which $R_1$ represents the radical:

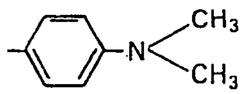

$R_2$ represents the radical $CH_3$ and

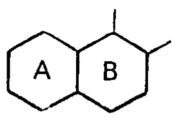

represents the radicals:

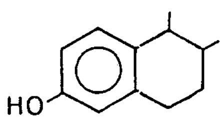 ; 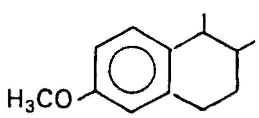 ;

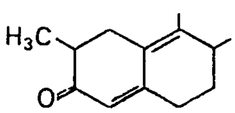 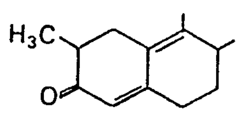

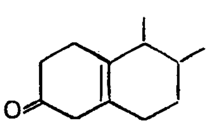 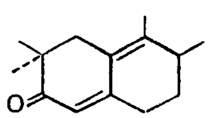

4. Process for the preparation of the products of the general formula $I_D$ as defined in claim 1 characterized in that:

a) In order to prepare the products of formula $I_{DA}$:

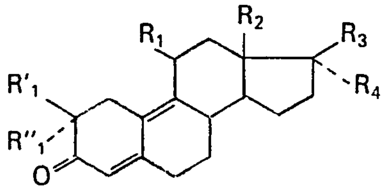

($I_{DA}$)

in which $R_1$, $R_2$, $R_3$ and $R_4$ retain the same meaning as in claim 1 and $R_1'$ and $R_1''$ are such that, either $R_1'$ and $R_1''$ each represent an alkyl radical or one represents a hydrogen atom and the other represents an alkyl radical or $R_1'$ and $R_1''$ each represent a nitrile radical or one represents an alkyl radical and the other represents a nitrile radical, a product of formula $II_D$:

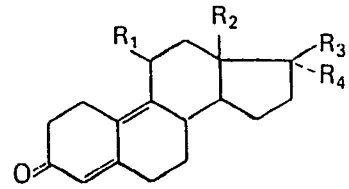

($II_D$)

in which $R_1$, $R_2$, $R_3$ and $R_4$ retain the same meaning as in claim 1, is submitted first optionally to the action of a protection reagent of the functional groups then to the action of a strong base and either of an alkyl halogenide or of tosyl cyanide, or first ot the action of an alkyl halogenide then of tosyl cyanide, then, if necessary, the protector group is eliminated;

b) 1) In order to prepare the products of formula $I_{DB}$:

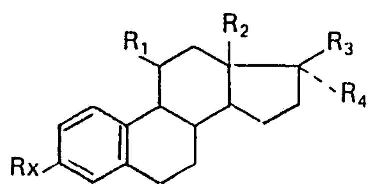

($I_{DB}$)

corresponding to the formula $I_{DB1}$:

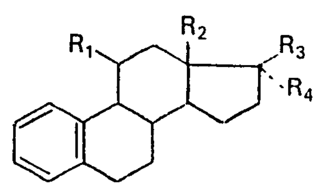

($I_{DB1}$)

in which $R_1$, $R_2$, $R_3$ and $R_4$ keep the same meaning as in claim 1, a product of formula $II_D$ is submitted to the action of a reducing agent, to obtain a product of formula $II_{D1}$:

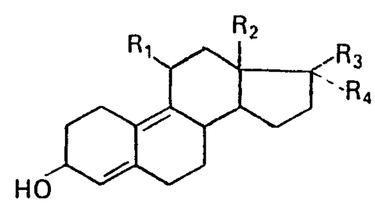

($II_{D1}$)

in which $R_1$, $R_2$, $R_3$ and $R_4$ keep the same meaning as above, which product is submitted to the action of an acid aromatization agent;

b) 2) in order to prepare the products of formula $I_{DB}$ corresponding to the formula $I_{DB2}$:

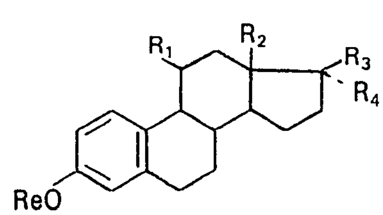

($I_{DB2}$)

in which $R_1$, $R_2$, Re, $R_3$ and $R_4$ keep the same meaning as above, a product of formula $II_D$ is submitted to the action of an aromatization agent then a saponification agent, then optionally to an alkylation or acylation reagent;

c) in order to prepare the products of formula $I_{DC}$:

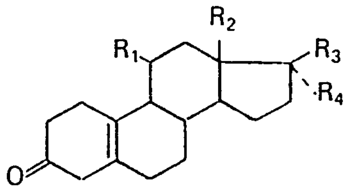

($I_{DC}$)

in which $R_1$, $R_2$, $R_3$ and $R_4$ keep the same meanings as in claim 1, a product of formula $II_D$ is submitted to the action of a reducing agent;

d) in order to prepare the products of formula $I'_{DA}$

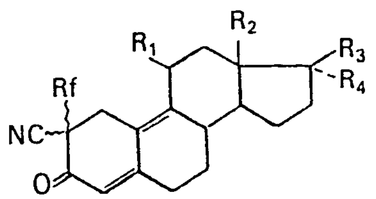

($I'_{DA}$)

in which Rf repesents a hydrogen atom or an alkyl radical and $R_1$, $R_2$, $R_3$ and $R_4$ keep the same meaning as in claim 1, a base is made to act on the products of formula $I_{DE}$ to obtain the products of formula $I'_{DA}$ in which Rf represents a hydrogen atom and, if desired, the products so obtained are submitted to the action of a strong base and an alkyl halogenide to obtain the products of formula $I'_{DA}$ in which Rf represents an alkyl radical.

5. The products of formula $II_D$:

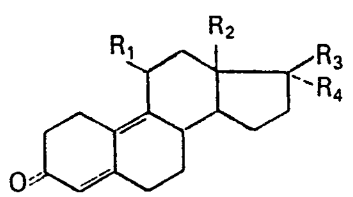

($II_D$)

in which $R_1$, $R_2$, $R_3$ and $R_4$ keep the same meanings as in claim 1.

6. Product of formula $II_D$ as defined in claim 5 in which $R_2$ represents a methyl radical and $R_1$ represents a radical:

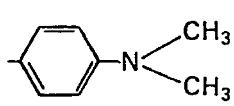

7. As medicaments, the products of formula I as defined in any one of claims 1 to 3.

8. The pharmaceutical compositions containing at least one of the medicaments according to claim 7 as active principle.

**Claims for the Contracting State: AT**

1. Process for the preparation of the products of the general formula $I_D$:

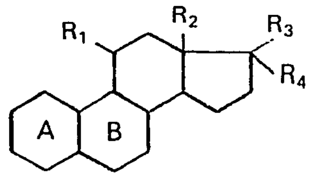

($I_D$)

in which $R_1$ represents an organic radical containing from 1 to 18 carobn atoms and optionally one or more heteroatoms, the atom immediately next to the carbon in position 11 being a carbon atom, $R_2$ represents a hydrocarbon radical containing from 1 to 8 carbon atoms, the rings A and B having one of the following structures:

a) – Either A and B represent the group:

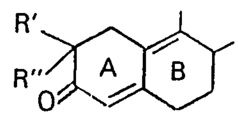

in which R' and R'', identical or different, represent a hydrogen atom, a nitrile radical or an alkyl radical having from 1 to 4 carobn atoms, it being understood that at least one of the radicals R' or R'' does not represent a hydrogen atom;

b) – Or A and B represent the group:

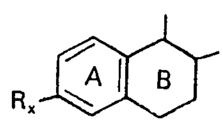

Rx representing a hydrogen atom or a –ORe group, in which Re represents a hydrogen atom, an alkyl radical having from 1 to 6 carbon atoms optionally substituted or an acyl radical;

c) – Or A and B represent the group:

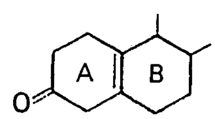

it being understood that when A and B represent the group

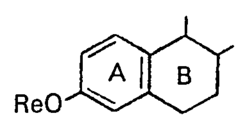

the radical $R_1$ contains at least one nitrogen, phosphorus or silicon atom, and that when A and B represent the group

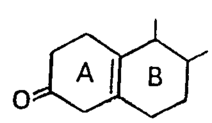

the radical $R_1$ does not represent a saturated linear alkyl radical, as well as the addition salts or the products of formula I with acids, and $R_3$ and $R_4$ together form the radical:

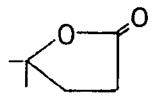

characterized in that:

a) to prepare the products of formula $I_{DA}$:

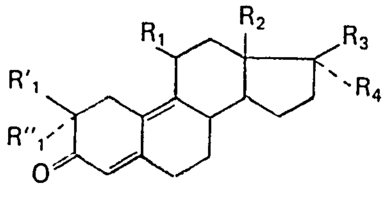

($I_{DA}$)

in which $R_1$, $R_2$, $R_3$ and $R_4$ keep the same meaning as above and $R_1'$ and $R_1''$ are such that, either $R_1'$ and $R_1''$ each represent an alkyl radical or one represents a hydrogen atom and the other represents an alkyl radical or $R_1'$ and $R_1''$ each represent a nitrile radical or one represents an alkyl radical and the other represents a nitrile radical, a product of formula $II_D$:

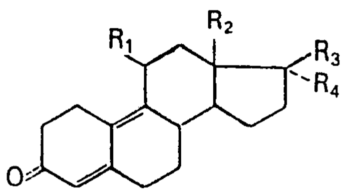

($II_D$)

in which $R_1$, $R_2$, $R_3$ and $R_4$ keep the same meaning as above, is submitted first optionally to the action of a protection reagent of the functional groups then to the action of a strong base and either of an alkyl halogenide or of tosyl cyanide, or first to the action of an alkyl halogenide then of tosyl cyanide, then, if necessary, the protector group is eliminated;

b) 1) In order to prepare the products of formula $I_{DB}$:

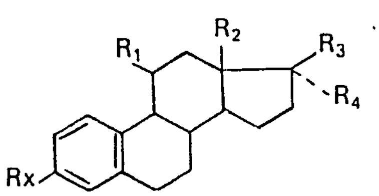

($I_{DB}$)

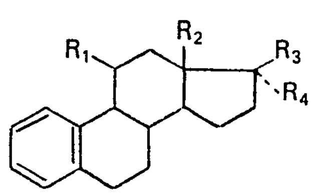

($I_{DB1}$)

in which $R_1$, $R_2$, $R_3$ and $R_4$ keep the same meaning as above, a product of formula $II_D$ is submitted to the action of a reducing agent, to obtain a product of formula $II_{D1}$:

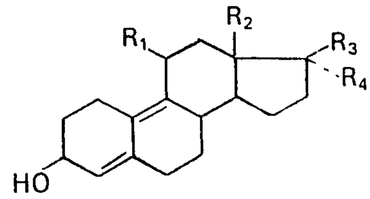

($II_{D1}$)

in which $R_1$, $R_2$, $R_3$ and $R_4$ keep the same meaning as above, which product is submitted to the action of an acid aromatization agent;

b) 2) in order to prepare the products of formula $I_{DB}$ corresponding to the formula $I_{DB2}$:

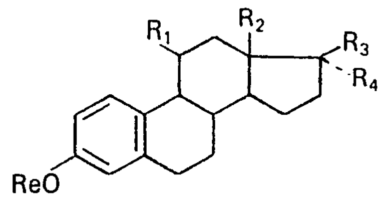

($I_{DB2}$)

in which $R_1$, $R_2$, Re, $R_3$ and $R_4$ keep the same meaning as above, a product of formula $II_D$ is submitted to the action of an aromatization agent then a saponification agent, then optionally to an alkylation or acylation reagent;

c) in order to prepare the products of formula $I_{DC}$:

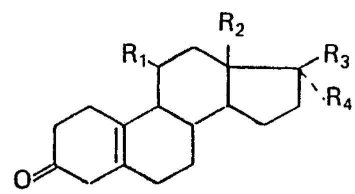

($I_{DC}$)

in which $R_1$, $R_2$, $R_3$ and $R_4$ keep the same meanings as above, a product of formula $II_D$ is submitted to the action of a reducing agent;

d) in order to prepare the products of formula $I'_{DA}$

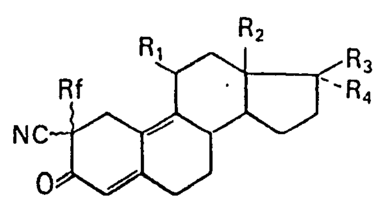

($I'_{DA}$)

in which Rf represents a hydrogen atom or an alkyl radical and $R_1$, $R_2$, $R_3$ and $R_4$ keep the same meaning as in claim 1, a base is made to act on the products of formula $I_{DE}$ to obtain the products of formula $I'_{DA}$ in which Rf represents a hydrogen atom and, if desired, the products so obtained are submitted to the action of a strong base and of an

alkyl halogenide to obtain the products of formula $I'_{DA}$ in which Rf represents an alkyl radical.

2. Process according to claim 1 characterized in that the products of formula $I_D$ are prepared, in which $R_1$ represents an aryl or an aralkyl radical bearing an amine function:

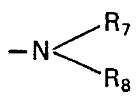

in which $R_7$ and $R_8$ represent an alkyl radical containing from 1 to 8 carbon atoms or a primary, secondary or tertiary radical containing from 1 to 8 carbon atoms, containing one or more heteroatoms selected from the group constituted by oxygen, nitrogen, sulphur and silicon of which there is at least one nitrogen atom, or substituted by a heterocycle containing at least one nitrogen atom.

3. Process according to claims 1 or 2 characterized in that the products of formula $I_D$ are prepared, in which $R_1$ represents the radical:

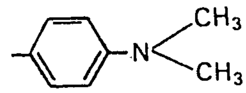

$R_2$ represents the radical $CH_3$ and

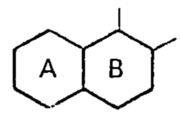

represents the radicals:

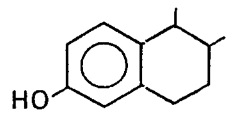 ; 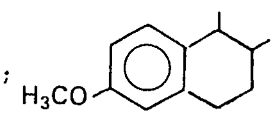 ;